# EUROPEAN PATENT SPECIFICATION

(11) **EP 1 546 328 B1**
(45) Date of publication and mention of the grant of the patent: **26.04.2006**
(21) Application number: 03776861.1
(22) Date of filing: 06.10.2003
(51) Int. Cl.: C12N 15/09, C12N 15/11, C12N 15/85, C12N 15/90, C12N 5/10, C12P 21/00

(54) **HIGH YIELD HETEROLOGOUS EXPRESSION CELL LINES FOR EXPRESSION OF GENE PRODUCTS WITH HUMAN GLYCOSYLATION PATTERN**
ZELLLINIEN ZUR HETEROLOGEN EXPRESSION VON GENPRODUKTEN MIT HUMANEM GLYKOSILIERUNGSMUSTER
LIGNEES CELLULAIRES D'EXPRESSION HETEROLOGUE A HAUT RENDEMENT POUR L'EXPRESSION DE PRODUITS GENIQUES PRESENTANT UN MOTIF DE GLYCOSYLATION HUMAIN

(30) Priority: 04.10.2002 EP 02022194
(43) Date of publication of application: 29.06.2005
(73) Proprietor: ProBioGen AG, 13086 Berlin (DE)
(72) Inventor: SANDIG, Volker, PROBIOGEN AG, 13086 Berlin (DE); WINKLER, Karsten, PROBIOGEN AG, 13086 Berlin (DE); MARX, Uwe, PROBIOGEN AG, 13086 Berlin (DE); WERMELINGER, Tobias, CH-8820 Wädenswil (CH)
(74) Representative: von Kreisler Selting Werner
(86) International application number: PCT/EP2003/011027
(87) International publication number: WO 2004/031383

(56) References cited:
- WO-A-00/63410
- WO-A-02/08409
- ESPERET CORINNE ET AL: "Non-erythroid genes inserted on either side of human HS-40 impair the activation of its natural alpha-globin gene targets without being themselves preferentially activated." JOURNAL OF BIOLOGICAL CHEMISTRY, vol. 275, no. 33, 18 August 2000 (2000-08-18), pages 25831-25839, XP002225659 ISSN: 0021-9258
- TRINH K R ET AL: "Site-specific and directional gene replacement mediated by Cre recombinase" JOURNAL OF IMMUNOLOGICAL METHODS, ELSEVIER SCIENCE PUBLISHERS B.V.,AMSTERDAM, NL, vol. 244, no. 1-2, 20 October 2000 (2000-10-20), pages 185-193, XP004218458 ISSN: 0022-1759 cited in the application
- KARREMAN S ET AL: "ON THE USE OF DOUBLE FLP RECOGNITION TARGETS (FRTS) IN THE LTR OF RETROVIRUSES FOR THE CONSTRUCTION OF HIGH PRODUCER CELL LINES" NUCLEIC ACIDS RESEARCH, OXFORD UNIVERSITY PRESS, SURREY, GB, vol. 24, no. 9, 1 May 1996 (1996-05-01), pages 1616-1624, XP000616161 ISSN: 0305-1048 cited in the application
- FENG Y-Q ET AL: "SITE-SPECIFIC CHROMOSOMAL INTEGRATION IN MAMMALIAN CELLS: HIGHLY EFFICIENT CRE RECOMBINASE-MEDIATED CASSETTE EXCHANGE" JOURNAL OF MOLECULAR BIOLOGY, LONDON, GB, vol. 292, no. 4, 1999, pages 779-785, XP000929622 ISSN: 0022-2836 cited in the application -& WO 00 63410 A (EINSTEIN COLL MED ;LEBOULCH PHILIPPE (US); MASSACHUSETTS INST TECH) 26 October 2000 (2000-10-26)
- FUSSENEGGER M ET AL: "Genetic optimization of recombinant glycoprotein production by mammalian cells" TRENDS IN BIOTECHNOLOGY, ELSEVIER, AMSTERDAM, NL, vol. 17, no. 1, January 1999 (1999-01), pages 35-42, XP004155532 ISSN: 0167-7799 cited in the application

## Description

### Technical Field

The present invention relates to ubiquitous/universal processes for establishing cells capable of stable high yield expression of a recombinant gene with human glycosylation pattern, and for establishing stable universal precursor cells available for insertion of arbitrary target genes. The invention further relates to the cells obtainable by said processes.

### Background of the Invention

Recombinant protein production is of central importance for different applications. Structural studies of proteins (rational drug design and drug optimisation are based thereon (Antivir. Chem. Chemother., 12 Suppl. 1, 43-49 (200:1))), industrial applications of proteins (enzymes) and clinical use of recombinant proteins have increased the need for their efficient production. As of February 2000, according to a survey by the Pharmaceutical Research and Manufacturers of America, 122 biologics, including 20 monoclonal antibodies were either in phase III trials or awaiting FDA approval (K. Garber, 2001, Nature Biotech. 19, 184-185).
Depending on the application, native conformation and correct posttranslational modifications (such as glycosylation) of the recombinant protein are essential. Prokaryots such as the biotechnology "pet" organism *Escherichia coli* (*E. coli*) lack the ability to introduce posttranslational modification. Only eukaryotic cells possess the cell machinery necessary for co-translational and post-translational modifications as they are often required to produce functionally active proteins. Various eukaryotic systems for the production of a variety of heterologous proteins exist. Fungal expression systems, using e.g. derived from the genus *Saccharomyces, Candida, Pichia, Hansenula, Aspergillus* or *Kluyveromyces* are well established (Hollenberg and Gelissen (1997), Current Opinion in Biotechnology 8, 554-560). To circumvent the problem of plasmid instability sometimes encountered in fungi, sequences coding for heterologous proteins are ideally integrated into the fungal chromosome via homologous recombination. Further problems encountered with fungal expression systems are overglycosylation of heterologous proteins and incorrect folding such as incorrect oligomerisation and insufficient ligand incorporation. Expression of heterologous proteins in insect cells - the DNA encoding the heterologous protein can also become integrated into the chromosome via recombination - gets around these problems. However, insect cells lack the ability to produce sialic acid and sialic glycans. Terminal sialic acid residues play divers biological roles in many glycoconjugates. Plants can also be used for the production of recombinant proteins. However, in these heterologous expression system difficulties in extraction and purification prove real bottlenecks.

Mammalian expression system, cultured cells as well as transgenic animals have none of these disadvantages. However, the posttranslational modifications vary between the mammalian species. Thus, the expression of human proteins in rodent cells (e.g. as disclosed in Sun, W. et al., J. Immunol. 152, p. 695-704 (1994); Lang, P. and Mocikat, R., Mol. Gen. Genet. 424, p 528-538 (1994); WO 96/30498, and Fukuta, K. et al., Arch. Biochem. Biophys. 378, p. 142-150 (2001) results in a human protein with rodent glycosylation pattern.

Recombinant proteins can be produced in cultured mammalian cells either transiently or constitutively (stably). For transient expression of recombinant a vector DNA encoding the recombinant protein is introduced into the cell and in general is not integrated into the cellular DNA. Expression titers of the recombinant protein are at the beginning high. However, since the vector DNA is generally not replicated, the vector DNA becomes diluted with each cell proliferation and hence the expression titer drops. Only rarely a vector DNA or part of the vector DNA illegitimedly recombines with the cellular genomic DNA and the gene encoding recombinant protein is stably integrated into the genome. If the gene encoding the recombinant protein is associated with a selection marker, cells carrying this cassette can be identified and isolated as stably transformed cells. Stable transformants have the advantage that the heterologous proteins are continuously produced. The expression titer is mainly determined by the strength of the promoter construct, the site of integration into the chromosome, the copy number and the type of recombinant protein in question. Many strong promoters are commercially available, however, their transcriptional activity varies depending on the cellular level of the relevant transcription factors and on the chromatin structure at the integration site. For example integration within the scaffod- or matrix attachment regions (S/MAR elements) of chromosomal DNA can augment the activity of promoters -and hence the expression of heterologous genes- and protect them from inactivation by the flanking chromatin. Therefore, it is highly desirable to chose a promoter highly active in a specific cell and to direct integration into an active part of the chromosome. Preferentially a single integration event is desirable, since heterologous genes at low copy number are in general expressed more stable than multicopy genes.

Integration at a single preselected highly active locus can be achieved via homologous recombination. This method, although typically applied to mouse embryonic stem cells, is extremely inefficient in somatic cells of human origin and requires a large scale screening effort. Moreover it is not applicable for most human permanent cell lines when it is desired to completely shut off the expression of a given target gene, because these cell lines are usually polyploid and targeting more than 2 identical loci is hardly feasible. Site specific recombination using recombinases, eg. Cre, flp, C13 and their respective target site (RRS) are a viable alternative (Feng, Y.Q. et al., Journal of Molecular Biology, vol. 292(4), p. 779-785 (1999); Schlake, T. et al., Biochemistry, Am. Chem. Soc., vol. 244(1-2), p. 185-193 (October 2000); Fussenegger, M. et al., Trends in Biotechnology, vol. 17(1), p. 35-42 (January 1999); Groth, A.C. et al., Proceedings of the National Academy of Sciences of USA, vol. 97(11), p. 5995-6000 (May 2000)). With this approach, a plasmid carrying a single RRS can be used to target a single RRS in the chromosome. This method, however, has certain limitations: Namely, it is quite inefficient because the reverse reaction, excision of the plasmid, is an intermolecular recombination and takes place at much higher speed than the integration. Secondly, the whole plasmid including bacterial genes are integrated. To solve the first problem unidirectional was established, e.g. by meains of heterospecific target sites for both flp and cre. These RRS are recognised by the respective recombinase but a successful recombination requires identical sites and the excision reaction is precluded (Karreman S. et al., Nucleic Acids Res., vol 24(9), p. 1616-1624 (1996); Trinh, K.R. et al., J. of Immunol. Methods, vol. 244, p. 185-193 (2000)). However, the targeting plasmid still has to be integrated into a single favourable position of the chromosome. A large scale screening effort is required to find such rare integrates. These clones often contain more than one copy of the plasmid, the may contain incomplete copies and bacterial sequences care not precluded from integration. These bacterial sequences are recognized by the mammalian cell often leading to inactivation of the targeted region. Alternatively, the targeting cassette may be integrated via retroviral vectors (Karreman S. et al., Nucleic Acids Res., vol 24(9), p. 1616-1624 (1996)). These vectors target active sites within chromosome, only full length cassettes are integrated and the infection dose can be adjusted to create single integration sites. However, expression units flanked by ITRs may also be subject to inactivation. In addition, the use of this system may be restricted by the governmental release agencies to exclude therapeutic applications of the expressed protein. Finally, Karpas A. et al., PNAS 98(4), 1799-1804 (2001) discloses a human myeloma cell line suitable for the generation of hybridomas that stably express immunoglobulins at high yield. On the other hand, is mentioned that myeloma are generally not suitable for that purpose.

### Summary of the Invention

In view of the above, there is still a need for a method allowing the transformation/conversion of a cell line with an arbitrary gene coding for a product of interest to obtain a high yield recombinant human glycoprotein producing cell, especially for a method without or only little cumbersome screening procedures. It was surprisingly found that cells expressing recombinant glycoproteins with features of human posttranslational modification at high yield are obtainable by first identifying a non-essential highly expressed cellular gene (hereinafter shortly referred to as "starting gene") in a human or essentially human hybrid cell (hereinafter shortly referred to as "starting cell"); secondly directly replacing the starting gene via homologous recombination with a first functional DNA sequence (e.g. by utilizing an appropriate targeting cassette) containing recombinase recognition sites (RRSs) for site-directed integration and optionally a "place-holder" gene comprising various functional sequences and selecting/isolating a stable clone of this precursor expression cell (functionalized cell); thirdly introducing the gene of interest (from here on called "target gene") coding for the target gene product (protein) by site-directed integration using a recombinase recognizing the RRSs incorporated with the first targeting cassette; and finally selecting/isolating a stable expression cell capable of producing large amounts of the recombinant protein. Direct replacement of the starting gene with a functional DNA sequence containing a DNA sequence coding for the target gene product is also applicable.

It was moreover found that suitable starting cells for the above method are specific mammalian cells such as human myeloma and hybridoma cells and human heterobybridoma cells (including human-mouse hetero-hybridoma cells such as H-CB-P1), which allow the production of proteins having an essentially human glycosylation pattern.

Using the present invention it is possible to introduce stably any gene encoding a recombinant protein of interest into the specific mammalian cells set forth above. Using the present invention the gene of interest encoding the recombinant protein will become integrated into the locus of a highly expressed cellular gene and preferably in close proximity to a highly active cellular promoter residing in an active part of the chromosome. Using the present invention precursor cell lines of various origin can be created carrying a place holder gene surrounded by RRSs. Using the present invention the place holder gene can be exchanged with the gene of interest, encoding the recombinant protein, by site-specific recombination at the RRSs catalyzed by a suitable recombinase, giving rise to the final high-yield expression cell.

Finally, it was found that the human-mouse heterohybridoma provides for a very distinct human glycosylation pattern.

More specifically, the present invention provides
(1) a process for preparing cells capable of stable high yield expression of a target gene product having essentially human glycosylation pattern which method comprises
   (a) selecting an immortalized human cell or human hybrid cell (hereinafter "starting cell") which is derived from B lymphocytes and is capable of stable high yield expression of a starting gene product being non-essential to the starting cell;
   (b) screening for the locus of the starting gene product within the genome of the starting cell;
   (c1) replacing the gene coding for the starting gene product with a first functional DNA sequence containing one or more recombinase recognition sites (RRS) to obtain a functionalized precursor cell; and
   (d) integrating a second functional DNA sequence containing a DNA sequence coding for the target gene product into the functionalized precursor cell obtained in step (c1) by use of a recombinase recognizing the RRSs incorporated with the first functional sequence, or
   (c2) directly replacing the gene coding for the starting gene product with a functional DNA sequence containing a DNA sequence coding for the target gene product;
(2) in a preferred embodiment of the process of (1) above the starting cell is a human-mouse hetero-hybridoma such as hetero hybridoma H-CB-P1 (DSM ACC 2104)) and integration of the functional DNA sequence(s) is effected at a Ig locus (preferably at a rearranged human Ig locus of said cell);
(3) a cell capable of high yield expression of a target gene product obtainable by the process of (1) or (2) above;
(4) a process for preparing a functionalized cell comprising the steps (a) to (c1) as defined in (1) or (2) above;
(5) a precursor cell obtainable by the process of (4) above; and
(6) a method for high yield expression of a target gene product which comprises cultivating a cell as defined in (3) above.

### Description of the Figures

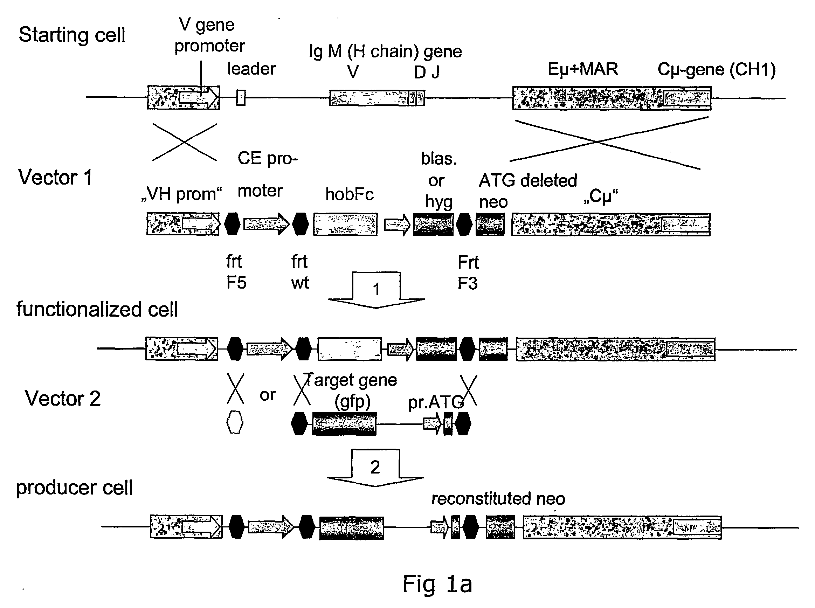

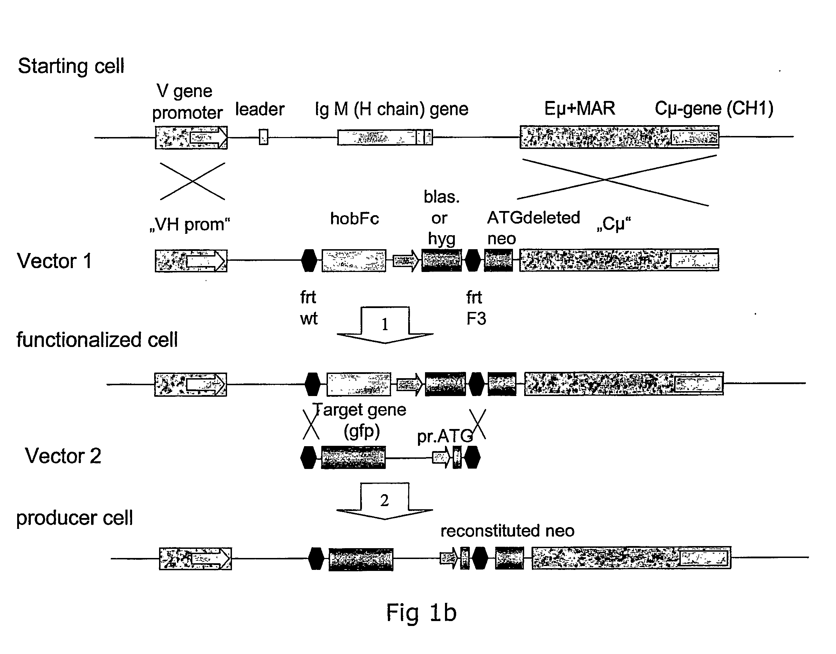

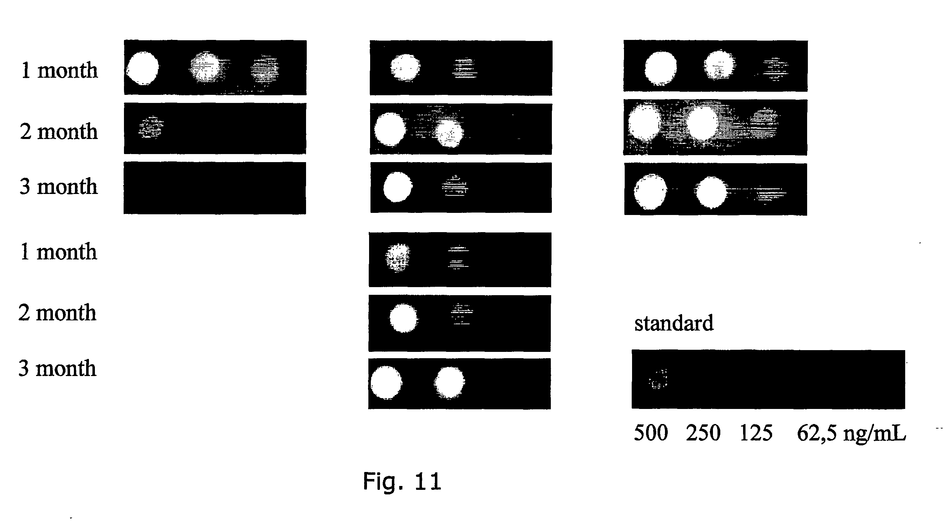

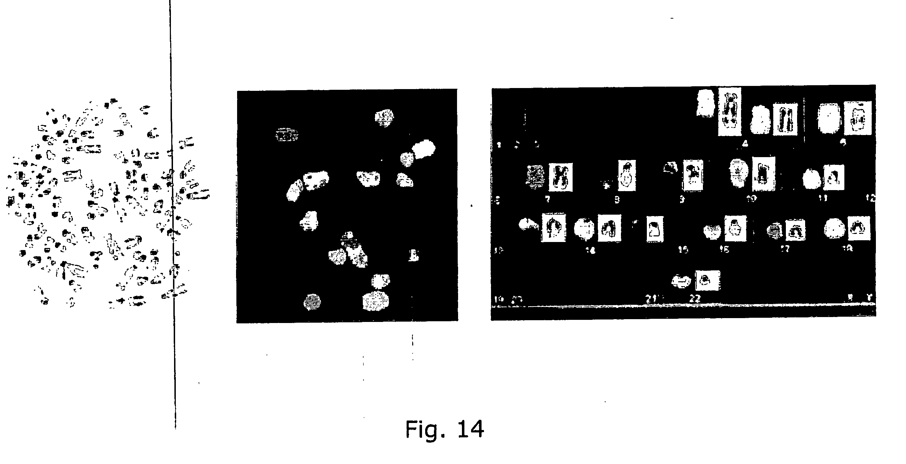

Fig. 1: Concept overview, multistep process to create high yield expression cell lines comprising site-specific integration of genes into an IgH locus at frt sites. The IgH locus of H-CB-P1 is represented in the upper graph of figures 1a and 1b. It contains the variable gene promoter followed by a protein leader sequence and specific V, D, and J genes rearranged and positioned next to the enhancer Eµ, the MAR and the Cµ coding sequences. Target sequences for homologous recombination are shown marmorate. Via homologous recombination between the flanking sequence elements "Vhprom" and "Cµ" of vector 1 (targeting vector) and the genomic DNA, first functionalized sequences located between the flanking sequences are introduced into the genomic DNA and a recombinant PBG03 genome is the result. The first functionalized sequence contains frt sites (frtF5 frtF3 and frt wt), an artifical strong promoter CES (Fig. 1a) or no additional promoter (Fig. 1b) upstream of the first expressed gene(hobFc). In addition a blasticidine or hygromycin resistance gene and an ATG deleted neomycin gene are part of the first functionalized sequence. The recombinant genome carries the first functional sequences integrated in the chromosomal DNA.
   FLP recombinase catalyses recombination at frtF5 and frtF3 or at frt wt and frtF3 sites of the recombinant H-CB-P1 genome and vector 2, the actual gene of interest is introduced and expressed from the artificial or the endogenous VH promoter (Fig. 1a and 1b, respectively). Parts of the first functionalized sequence located between frtwt and frt F3 sites are replaced by a weak promoter followed by an ATG which after recombination is positioned in the same open reading frame as the ATG-deleted neomycin gene. The resulting genome has lost the hobFc gene and the blasticidin or hygromycin resistence genes and instead has the gene of interest (target gene) and a functional neo gene.

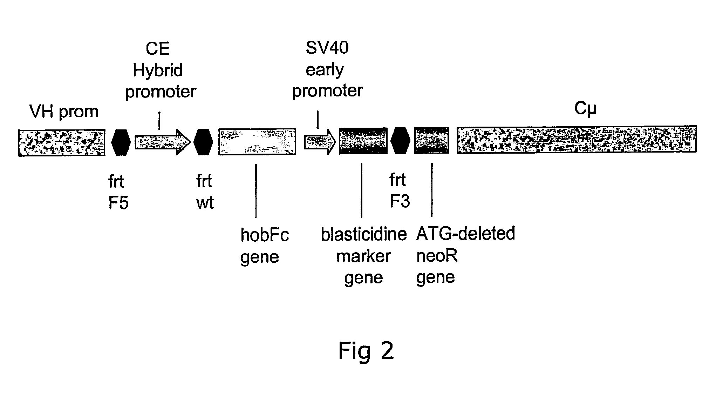

Figure 2: The endogenous cassette (CEShobFcblas) of the targeting vector.
   The detailed structure of the endogenous cassette containing an CES promoter, a hobFC fusion gene, a blasticidin resistance gene and a start codon (ATG) deficient neomycin gene is shown. In more detail, the endogenous sequence contains a modified frt site (frtF5) followed by a hybrid promoter structure comprising the early CMV promoter/enhancer elements as well as the first intron of the elongation factor alpha gene. The next element is a frt wildtype site followed by the hobFC fusion gene (hobFC), and the SV40 polyadenylation signal (SV40PA). A weak SV40 promoter controlling the expression of the blasticidin resistance gene follows. The last elements are a modified frt site (frtF3) and next to it the ATG deficient neo gene. Modified frt sites F3 and F5 allow recombination with identical sites but not with wildtype frt sites and F5 or F3 sites respectively. The frt F3 site is positioned to upstream of the neo gene to form a contiguous open reading frame lacking the ATG.

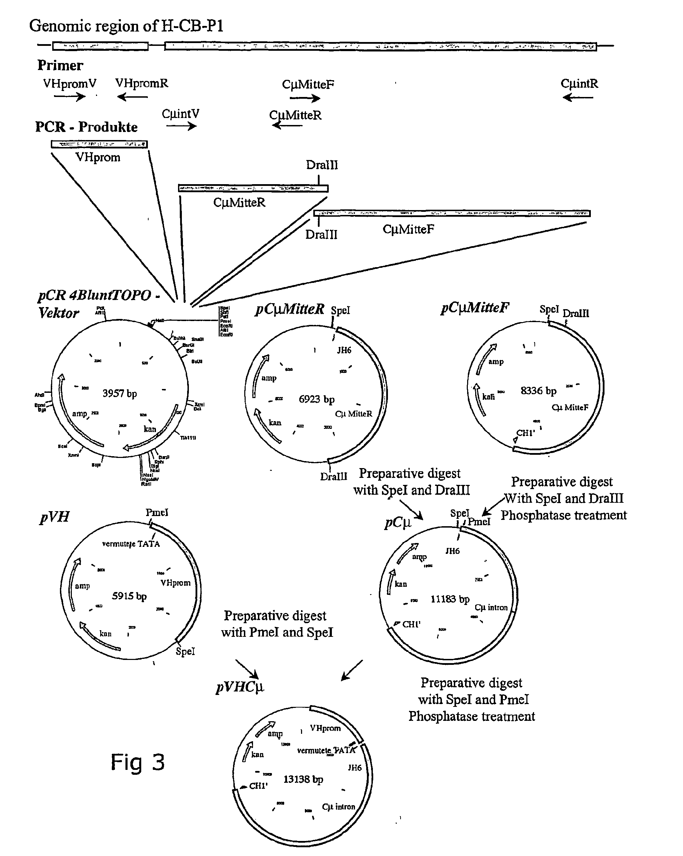

Fig. 3: Cloning strategy for the intermediate vector pVCµ containing the flanking regions Vhprom and Cµ.
   The 2 kb VH promoter sequence was amplified from PBG03 cell genomic DNA using forward primer VHpromF (SEQ ID NO:1) and reverse primer VHpromR (SEQ ID NO:2). The PCR product VHprom was cloned into the pCR 4BluntTOPO vector (Invitrogen) and the resulting vector named pVH. The 7.4 kb Cµ region was amplified from genomic H-CB-P1 DNA as two overlapping fragments, namely CµmitteR and CµMitteF. The primers CµintV (SEQ ID NO:3) and CµMitteR (SEQ ID NO:5) give rise to the product CµMitteR and the primers CµMitteF (SEQ ID NO:6) and the reverse primer CµintR (SEQ ID NO:4) produce the fragment CµMitteF. Both fragments were cloned into a pCR 4BluntTOPO vector (Invitrogen) and the resulting vectors called pCµMitteR and pCµMitteF. The full length Cµ sequence was re-established by opening both vectors with the restriction enzyme SpeI and DraIII, and ligating the SpeI-DraIII fragment from pCµMitteR into the opend pCµMitteF. The resulting vector pCµ carries the full-length Cµ region (Cµ intron). The VH promoter sequence and the Cµ sequence were combined in one vector pVHCµ, by digesting both pVH and pCµ with the restriction SpeI and PmeI, and inserting the isolated Vhprom PmeI-SpeI fragment into the phosphatase treated opend vector pCµ.

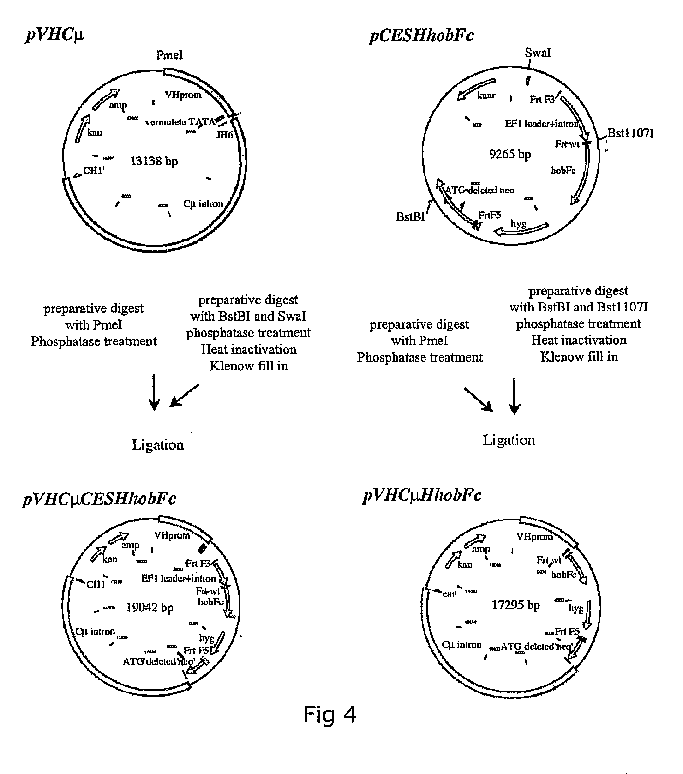

Fig. 4: Cloning strategy for the targeting vectors pCESHhobFc and pVHCµHhobFc. The targeting vector pVHCµCESHhobFc containging the highly active promoter CES and the hobFC fusion gene was created by ligating an end-filled SwaI-BstBI fragment isolated from pCESHhobFc into the pVHCµ vector that had been digested with PmeI and dephosphorylated. The targeting vector pVHCµHhobFc that has the hobFC fusion gene but no CES promoter, was prepared by ligating an endfilled Bst1107-BstBI fragment isolated from pCESHhobFc into an PmeI digested and dephosphorylated vector pVHCµ.

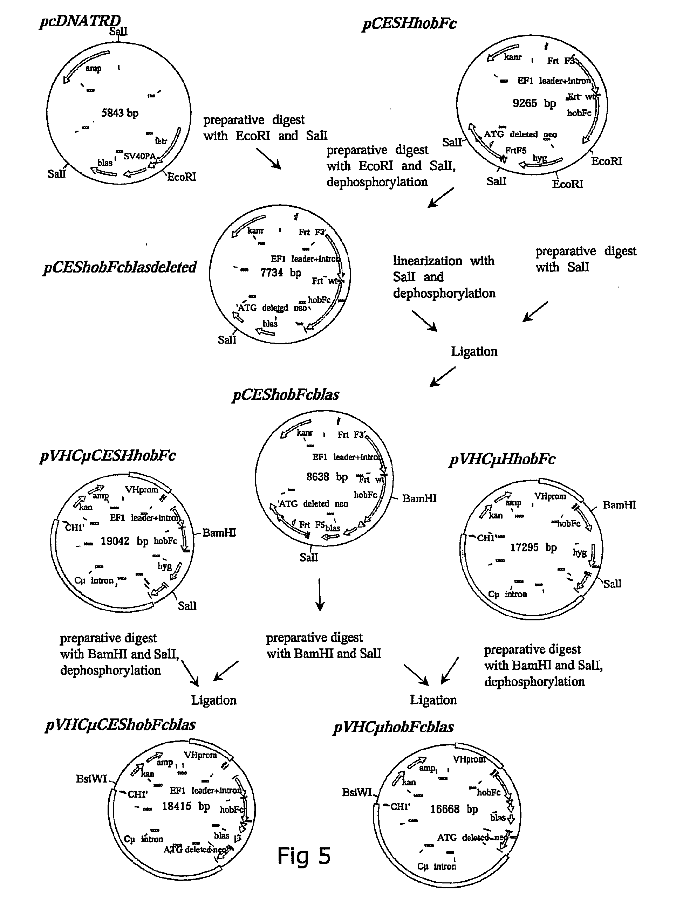

Fig. 5: Cloning strategy for targeting vectors carrying a blasticidin resistant gene, pVHCµCEShobFcblas and pVHCµhobFcblas.
   The plasmid pcDNATRD was used as donor for the blasticidin gene. To delete the hygromicin gene sequences as well as the FRT5 and neomycin sequences from the vector pCESHhobFc, the vector was digested with EcoRI and SalI and dephosphorylated. An EcoI-SalI fragment from pCDNATRD containing the blasticidin resistant gene sequence was ligated into the previously opened pCESHhobFc vector and the resulting plasmid named pCEShobFcblasdeleted. The Frt F5 sequence and the ATG-deleted neomycin sequence were isolated from pCESHhobFc as a SalI SalI fragment and re-inserted into the SalI site of pCEShobFcblasdeleted. The resulting plasmid pCEShobFcblas was used together with pVHCµCESHhobFc to create pVHCµCEShobFcblas. The BamHI-SalI fragment comprising the hobFc sequence and the blasticidin gene was isolated from pCEShobFcblas and inserted into vector pVHCµCESHhobFc opened with BamHI and SalI, giving rise to pVHCµCEShobFcbfas. The vector pVHCµhobFcblas was created by ligating a BamHI-SalI fragment containing the hobFc gene and the blasticidin gene into vector pVHCµHhobFc digested with BamHI and SalI.

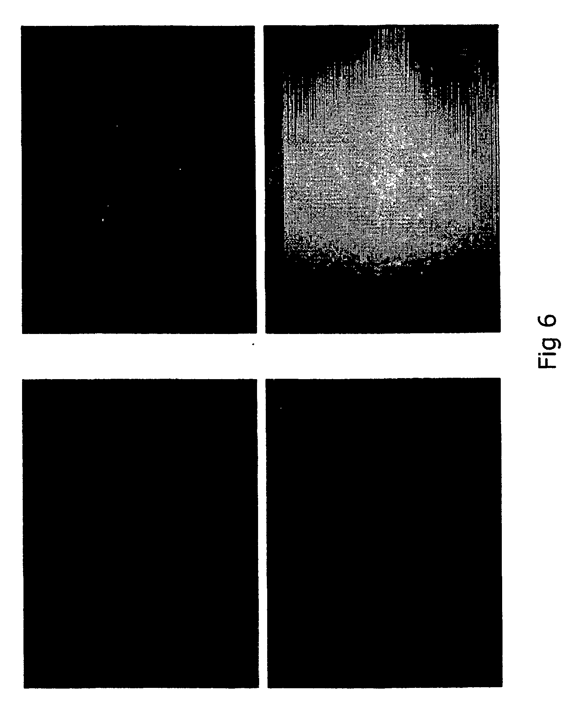

Fig. 6: Immunostaining of H-CB-P1 clones
   H-CB-P1 clones obtained through transfection of H-CB-P1 cells with pVHCµ CESHhobFcblas were immunostained with a Texas Red conjugated anti-human IgG, F_{y} fragments specific antibody isolated from goats or an AMCA conjugated anti-human IgM, Fc_{5µ} specific antibody isolated from goats. The left column shows two H-CB-P1 clones stained with the Texas Red conjugated antibody and visualized with an UV WG filter. In the right column the same clones are shown after staining with the AMCA conjugated anti-IgM antibody and visualized under UV filter WU. Whereas for the clone in the upper panel only IgG staining is evident, staining with both antibodies is present for the clone in the lower panel. The first clone may result from homologous recombination whereas the other clone contains an illegitimate insertion of the functional sequences.

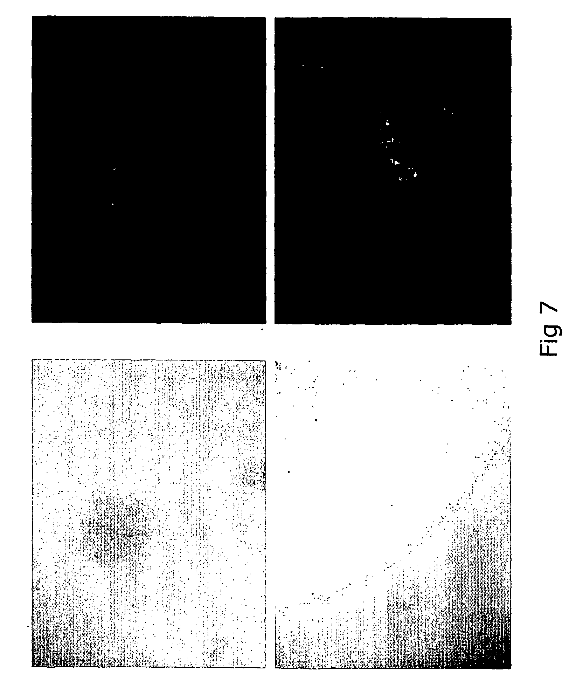

Fig. 7: Direct immunostaining of H-CB-P1 clones and further expansion of clones.
   It is demonstrated that H-CB-P1 clones could be immunostained without jeopardizing the cells viability and that subsequent expansion of the stained clone was possible. A H-CB-P1 clone cultured for ten days in a 96 well plate was immunostained with an Texas Red conjugated anti-IgG antibody. A picture of the clone prior to immunostaining visualized with normal light microscopy, is shown in the top left panel. The same clone immunostained with the Texas Red conjugated anti-IgG antibody is shown in the top right hand panel. The bottom panels show pictures of the well post trypsinisation. No cells can be seen when the picture was taken under a normal light microscope, as shown in the bottom left hand panel. The right hand panel shows the same well examined under UV filter WU. The cells are completely removed from the well and the fluorescent antibody precipitate remained in the well and is not attached to the cell surface.

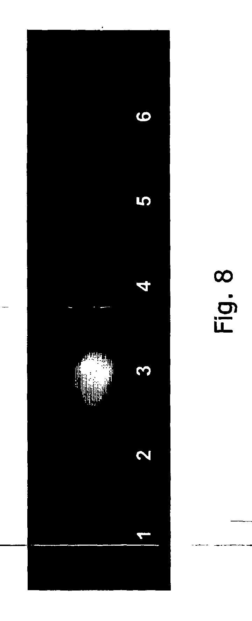

Fig. 8: Ant-IgM dot blot of cell culture supernatants from induvidual clones
   Supernatant of the following clones
   1: pVHCµhobFcblas-D6; 2: pVHCµhobFcblas-G8;
   3: pCEShobFcblas-A3; 4: pVHCµCEShobFcblas-B4;
   5: pVHCµCEShobFcblas-D3; 6: pVHCµCEShobFcblas-G8 were spotted onto a membrane and subjected to the ECR staining method. Since the starting cell population homogenously produces IgM, clones with no detectable IgM expression result from inactivation of the IgM H gene mediated by the targeting vector.
   The clone A3 generated by transfection of pCESHhobFcblas which lacks homologous flanking sequences was unable to target the IgM locus and expresses IgM.

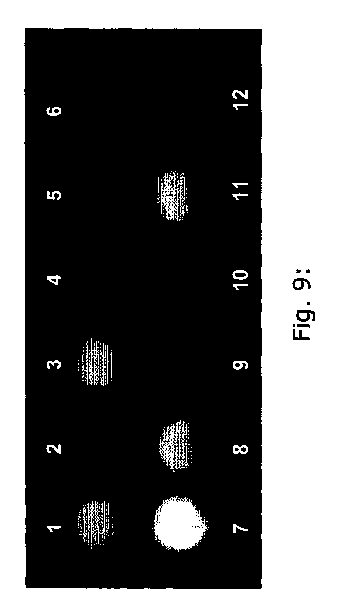

Fig. 9: Anti-IgG dot blot of cell culture supernatants from individual clones.
   Supernatant from the following clones
   1: pVHCµhobFcblas D6; 2: pVHCµhobFcblas D6 (1:2 diluted); 3: pVHCµhobFcblas-G8; 4: pVHCµhobFcblas G8 (1:2 diluted); 5: pCEShobFcblas A3; 6: pVHCµCEShobFcblas B4; 7: pVHCµCEShobFcblas D3; 8: pVHCµCEShobFcblas D3 (1:2 diluted);
   9: pVHCµCEShobFcblas D3 (1:10 diluted); 10: pVHCµCEShobFcblas G8;11:hobFc standard 500 ng/ml; 12: hobFc standard 50 ng/ml IgG
   were spotted onto a membrane and subjected to the ECR staining procedure using an anti-IgG antibody.

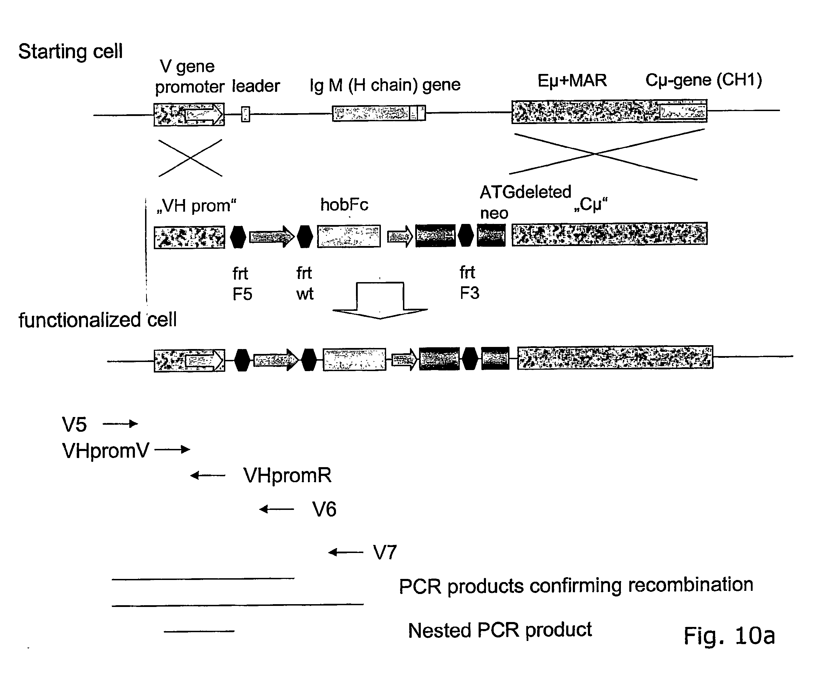

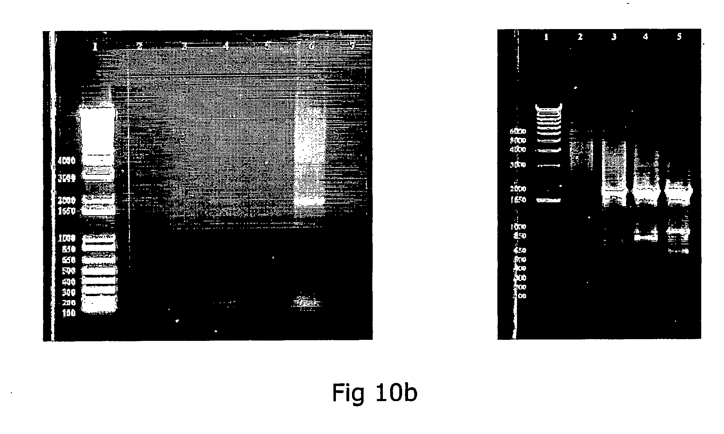

Fig. 10: Detection of homologue recombination via PCR
   To test whether a homologue recombination event had occurred between the (targeting) vector and the Ig locus of H-CB-P1 cells, a PCR strategy was applied. Upon recombination the endogenous cassette of the vector containing the CES promoter, the hobFc gene and a resistance gene (hygromycin or blasticidin) followed by the ATG-deleted neomycin gene_ becomes integrated between the genomic V gene promoter sequences and the enhancer Eµ. The forward primer V5 (SEQ ID NO:7) which binds to the genomic V gene promoter sequences outside of the fragment Vhprom was combined with primers V6 or V7 (SEQ ID NOs:8 and 9, respectively), which bind specifically within the first functional sequence. The occurrence of PCR products is strictly dependent on co-localisation of both primer binding sequences and hence homologous recombination. To confirm any positives the putative positiv PCR product was used in a nested PCR reaction with the primers VHpromF and VHpromR (SEQ ID NOs:1 and 2, respectively).
   a: primer position, b: electrophoretic analysis of PCR products
   left: first PCR V5, V7
      lane 1: 1kb ladder (Invitrogen); lane 2: clone pVHCµhobFcblas H6; lane 3: clone pVHCµCEShobFcblas B10; lane 4: clone pVHCµhobFcblas D4; lane 5: clone pVHCµhobFcblas D8; lane 6: clone pVHCµhobFcblas E11; lane 7: neg control H-CB-P1
   right: nested PCR
      lane 1: 1kb ladder (Invitrogen); lane 2: neg control H-CB-P1; lane 3: clone pVHCµCEShobFcblas H6; lane 4: clone pVHCµhobFcblas D4; lane 5 clone pVHCµhobFcblas E11
Fig. 11: hobFc expression in the absence of selction pressure.
   Cells were cultivated for 3 month in the absence of selection pressure. To determine expression cells were seeded at a density of 10⁵ cells/ml. After 24h cell culture supernatants were harvested and subjected to a westen blot (dot blot) using an anti human Fc antibody. Supernatants were applied to the filter from left to right: undiluted, 1:2 dilution and 1:10 dilution.
   Left: clone pCEShobFcblas A3 (random insertion),
   Center top pVHCµhobFcblas G8; Center bottom pVHCµhobFcblas G8;
   Right pVHCµCEShobFcblas D3.
   Expression is stable in clones resulting from homologous insertion of the functionalised sequences including the hobFc gene.

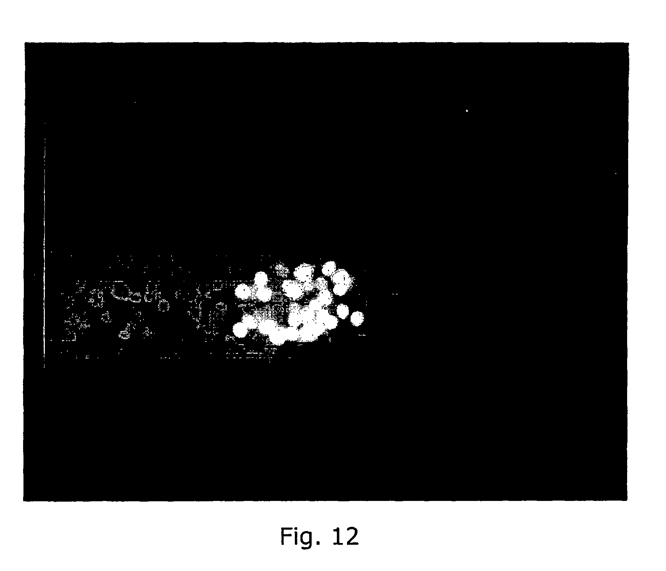

Fig 12: Generation of a target cell clone using GFP as a model target gene.
   Clone pVHCµCEShobFcblas D3 (renamed PBG04) was transfected with vector 2 comprising a second fuctional sequence (frtwt, GFP open reading frame and polyadenylation signal, minimal promoter followed by ATG and frtF5) and plasmid pflp comprising a functional expression unit for the flp recombinase (Note: the vector is unable to express GFP in a naive cell because it lacks a functional promoter driving expression). After two weeks of selection with G418 individual stable clones strongly expressing GFP are detectable. GFP expression as well as G418 resistance depends on the homologous recombination event.

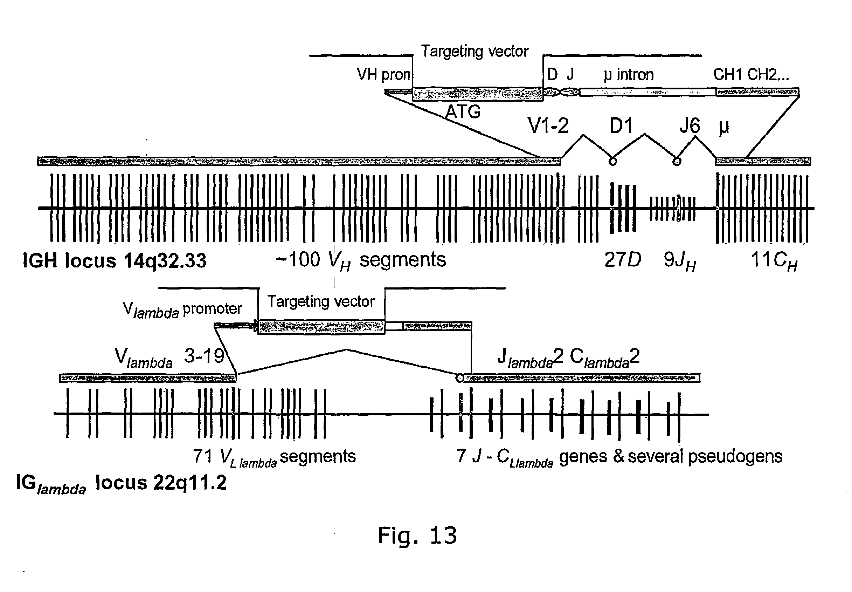

Fig. 13: Identification of the rearrangements in the heterohybridoma H-CB-P1
   The cDNA for the light and heavy chain genes form H-CB-P1 was sequenced and compared to database sequences. Genomic genes constituting the heavy gene genes were identified by homology with unrearranged genomic sequences. Based on the identification of V1-2, D1, J6 and µ a genomic map of the rearranged locus was constructed. PCR primers were designed using this information, respective fragments extending 5' to the variable gene V1-2 promoter and containing the D,J, and µ Intron sequences but leaving out the variable gene ATG were amplified and used to construct the targeting vector.
   The lambda light chain variable gene V3-19 was identified via homology search as well. This approach was not suitable to identify the constant gene because the locus contains 100% identical gene copies. A PCR based on primers in the intervening sequences between constant region genes allowed to identify J2 and H2 as the genes constituting the rearranged lambda gene of H-CB-P1
Fig. 14: Chromosome analysis of H-CB-P1. GTG Banding, left panel 68-94 chromosomes were fond. The majority were identified as mouse chromosomes Spectral Karyotype Analysis, middle and right panels. Human chromosomes within H-CB-P1 were identified by hybridisation with specifically labeled humanchromosome libraries. 8 intact human chromosomes 4, 5, 7, 10, 14, 17, 18, 22 In addition Chromosome fragments of ch. 4, 8, 9, 10, 11, 14, 16 were identified A hybridization with a probe specific for the human Ig H locus revealed a single IgH locus on the intact chromosome 14
Fig 15: Schematic representation of a N linked oligosacharide structure of a mammalian glycoprotein.
   The leptin-Fc molecule contains two N linked oligosacharides, one on each chain of the Fc domain.
Fig. 16: Aminophase-HPLC of leptinFc
   LeptinFc from PBG-04 was generated in roller bottle culture and purified by a generic process including affinity chromatography, gel filtration and membrane filtration. The protein was digested with trypsin and the resulting peptides were deglycosylated by PNGase F digestion. The glycans were labeled with 2-aminobenzamide and separated by HPLC on a Phenomenex Hypersil APS-2-column. Peak numbers represent the fractions which were used in MALDI-TOF-MS analysis.

### Detailed Description of the Invention

The present invention provides a process to transform a mammalian starting cell, in particular a human cell or human hybrid cell into a stable high yield expressing cell. To achieve continuous recombinant expression, the gene encoding the recombinant product becomes integrated into the genomic cellular DNA. Expression levels are highly determined by the site of integration of the recombinant gene into the cellular DNA. Therefore, the here presented method comprises the integration of a recombinant gene into a transcriptionally highly active part of the genome of a cell. The gene of interest coding for the recombinant protein can either be under the control of very strong recombinant promoter, or be placed under the control of a highly active cellular promoter by integrating it downstream of the highly active cellular promoter.

In a preferred embodiment of the process (1) of the invention the starting cell secretes the starting gene product, preferably in an amount of at least 0.3 fmol/cell/d of a polypeptide chain (which equals 30pg/cell /day for a protein of approximately 90kd) and more preferably in an amount of more than 1fmol/cell/d (which equals 100pg/cell /day for a protein of approximately 90kd). Alternatively, in case the starting cell does not secrete the starting gene product a gene coding for a highly expressed preferably nonessential intracellular or membrane protein or a highly expressed noncoding RNA is selected.

In another preferred embodiment, the starting cell is a primary, immortalized or fusionated cell or a genetic modification thereof. Thus, the starting cell may be selected from primary cells, immortalized cells (e.g. immortalized cells derived from B lymphocytes) or tumor cells or genetic modifications thereof, cell hybrids, cell lines used generally in protein manufacturing such as HEK293, PER.C6 human cell lines created from primary cells via genetic immortalization or fusion with immortal cell lines, preferably it is a human hybridoma or hetero-hybridoma cell (e.g. human-mouse, human-rat or the like) and most preferably is human-mouse hetero-hybridoma H-CB-P1 (DSM ACC 2104; previously referred to as ZIM517).

If the starting cell is a human cell or human heterohybridoma (e.g. as defined above), it is preferred that said hybrid cell or heteor-hybridoma comprises at least one human chromosome and/or is capable of human post-translational modification. It is particularly preferred that the starting gene product is a human gene.

The starting gene product is preferably selected from secreted proteins such as antibodies, cytokines, hormones, enzymes, transport proteins, storage proteins, and structural proteins. The starting gene product is either known a main product of the chosen starting cell. So stable expression of IgM has been observed for H-CB-P1 or selected in a screening procedure. Screening may be based on individual or combined methods comprising microarray expression analysis, 2D protein gel electrophoresis, quantitative PCR, RNAse protection, northern blot, ELISA and western blot. The power and sensitivity of these individual methods is known to those skilled in the art.

The method of the invention allows the production of any recombinant protein. Preferred target gene products include enzymes, in particular proteases, protease inhibitors, hormones, cytokines, receptors or soluble forms thereof (e.g. receptors lacking transmembrane or intracellular domains), full-length antibodies or antibody domains and fusion proteins combining domains of these protein classes.

In a first option of embodiment (1) comprising steps (a), (b), (c1) and (d), the replacement of the starting gene is effected by an one step replacement strategy, wherein the starting cell is contacted with a vector construct containing the first functional sequence, said first functional sequence inactivating and partially or completely replacing the gene coding for the starting gene product. Alternatively, the replacement is effected in a two or multistep strategy, wherein the gene coding for the starting gene product is deleted or inactivated and subsequently contacted with a vector containing the first functional sequence, said first functional sequence being incorporated at the site of the deleted/inactivated starting gene product.
Specific incorporation of the first functional sequence at the location of the starting genes is facilitated by sequences flanking the first functional sequence in the vector which are homologous to the target gene or adjacent sequences. These flanking sequences are obtained either from lambda, cosmid, pac or bac libraries of the starting cell or generated by PCR using starting cell DNA as a template.
The percentage of cell clones resulting from specific incorporation of the first functional, sequence at the location of the target gene may be further increased by employing a dual selection strategy, where a positive selection marker is contained as part of the first functional sequence and a negative selection marker separated from the first functional sequence by a homologous flank. Homologous exchange allows incorporation of the positive selection marker in the absence of the negative selection marker. Examples for positive selection markers are the hygromycin, blasticidin, neomycin, or glutamin synthetase genes and the HSV tk or the Cytosine desaminase gene are negative selection markers. Markers and methods for their application are known to those skilled in the art.
Cell clones resulting from homologous exchange are identified by the presence of elements of or gene products expressed from the first functional sequence and the inactivation of at least one allele of the starting gene. These cell clones represent the functionalised precursor cell.

The first functional sequence comprises one or more RRS(s) selected from loxP, frt, att L and attR sites of lambdoid phages, recognition sites for resolvases or phage C31 integrase. It is preferred that said recognition sites provide for unidirectional integration, which is achieved, e.g. by modified loxP and frt sites as well as by the (wild-type) recognition sites of ΦC31 integrase. The first functional sequence may further comprise sequences selected from marker sequences, secreted protein genes, promoters, enhancers, splice signals, polyadenylation signals and IRES elements.

To create a producer cell for the target gene product, the functionalized precursor cell (if not already a producer as obtained in step (c2) of second option of embodiment (1), see below), e.g.the PBG03 clone D3 (DSM ACC2577), is subsequently contacted with a second vector containing the second functional sequence. The second functional sequence comprises the target gene and RRS(s) for said recombinases present in the first functional sequence. The second functional sequence further comprises functional sequences selected from promoter sequences, marker sequences, splice donor and acceptor sequences and recombinase recognition sequences differing from RRS of the first functional sequence.

The integration of the second functional DNA sequence is effected by recombinases recognising the RRS with or without accessory proteins (e.g., Cre, Flp, φ C31 integrase and resolvase). These recombinase and accessory proteins, mRNA coding for these proteins or viral or nonviral vectors allowing there transient expression are delivered together with, shortly before or after delivery of the second functional sequence.

A pure population of clones containing the second functional sequence at the location of the starting gene is achieved by selection using a reconstituted functional selection marker gene. As an example an inactive ATG deleted selection marker gene introduced with the first functional sequence may be reconstituted by delivery of an active promoter and an in frame-ATG codon with the second functional sequence.

In a second option of embodiment (1) of the invention (comprising steps (a), (b) and (c2)) the gene coding for the starting gene product may directly (i.e. without the provision of the precursor cell) be replaced with a functional DNA sequence containing a DNA sequence coding for the target gene product (hereinafter shortly referred as "third DNA sequence"). Said third DNA sequence may be incorporated by a one- or multi-step strategy as described herein before. The third DNA sequence may further contain functional sequences (such as promoters and markers) as the first and second DNA sequence described herein before. This second option of embodiment (1) of the invention is particularly preferred, if only one target gene product is to be produced so that the generation of the precursor cell is not necessary.

In preferred embodiment (2) of the invention, the starting cell preferably is a human-mouse hetero-hybridoma cell, preferably is hetero-hybridoma cell H-CB-P1 (DSM ACC2104). The integration of the functional DNA sequence is effected at a Ig locus, preferably at one of the human rearranged Ig loci (e.g. heavy chain or light chain (λ, or κ)) of the hybridoma cell. The rearranged immunoglobin locus is the genomic sequence surrounding the functional Ig gene (heavy chain λ or κ) modified from the germ line chromosomal configuration during maturation of the B-lymphocyte which gave rise to the hybridoma. The IgH locus is located at chromosome 14q32.33. In H-CB-P1 this locus is formed by the rearranged and affinity matured VH1-2 gene linked via a D-gene to the J_{H}6-gene linked via the µ-intron to the Cµ sequences (DD 296 102 B3). The sequence of the rearranged VDJ region of the H-CB-P1-IgH locus is provided in SEQ ID NO:12.

It is preferred that the cells of embodiments (3) and (5) of the invention are derived from H-CB-P1 (DSM ACC2104). Furthermore, it is preferred in embodiment (3) of the invention that the target gene product is an antibody. In such case the cell is preferably PBG04 (DMS ACC2577). In the above cells in particular utilized for the expression of antibodies it is feasible that its light chains are inactivated (disrupted) or replaced with a gene coding for same or different target gene product.

Moreover, the target gene products obtainable by expression of a cell line derived from H-CB-P1 possesses a unique essentially human glycosylation pattern.

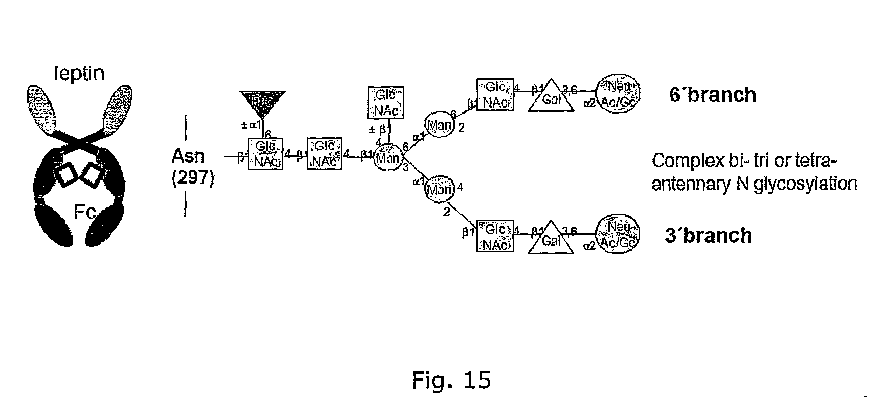

Glycoproteins for therapeutic application, in particular antibodies are typically manufactured in mammalian cells because posttranslational modifications such as N linked glycans are generated only in mammals and they have a substantial impact on pharmacological features of these proteins. A fully processed N glycan forms a biantennary structure with core fucose and terminal sialic acids (Fig 15). The majority of proteins, under physiologic conditions, carries only truncated versions of the full structure. The degree at which glycosylation is driven to completion depends on the cell type as well as on culture conditions.
So the degree of sialinisation the addition of the terminal sialic acid to the glycan varies and does rich only 30-40 % for antibodies in human blood. However, a high percentage of sialinated proteins increases the half life of a therapeutic protein in blood. The cell lines derived from H-CB-P1, such as PBG04 generate highly sialinated glycoproteins in comparison to CHO and NS0 cells widely used in the manufacture of glycoproteins as can be seen from Example 5. For therapeutic glycoproteins a low content of glycans terminated before the addition of galactose (G0 structures) is advantageous. So G0 Glycoproteins tend to dimerize and the ability of antibodies to mediate complement dependent cytotoxicitiy is diminished. The genetic composition of PBG04 allows a more complete processing with a low degree of G0 structures (4.3% on leptin-Fc in a roller bottle process)
Whereas the general biantennary structure is formed by all mammals, some specific structures (linkages between individual sugars) are either specific to, or completely excluded in humans. These structures affect biological features as well. Therefore, it is of advantage to use cells to manufacture glycoproteins for therapeutic applications which provide the necessary enzymes to generate human specific modifications and lack enzymes which are not present in human cells are responsible for atypical linkages. Such cells may be entirely human or contain a subset of human chromosomes. In the latter cells it is important that the human specific glycosylation enzymes dominate over those, not present in humans.
So neuraminic acid may be added as N acetylneuraminic acid or N glycolyl-neuraminic acid, the latter being the major structure in mouse cells. N glycolyl-neuranminic acid is absent on glycans form old word monkeys and man. They are immunogenic and may lead to the formation of antibodies against the therapeutic protein.
In addition, mouse cells contain an additional glycosylation enzyme, the alpha 1,3 galactosysltransferase. It mediates the transfer of gal residues to exposed gal residues of the glycan. Such linkage is also found in yeast and as a protection humans have pre-existing antibodies against this structure. Recognition may lead to the formation of immune complexes and kidney damage as a result of treatment. Only 1.3% PBG04 derived leptin-Fc contains alpha 1,3 gal.
A small percentage of human proteins contains bisecting N-acetylglycoseamine. It does not, per se, influence biologic features but the enzyme complex interferes with another one mediating core fucosylation. Often, in proteins with bisecting N-acetylglycoseamine, core fucose is missing, resulting in more efficient binding of the Fc-gamma Receptor and in enhancement of antibody dependent cellular cytotoxicity (ADCC). Therefore cells have been engineered to express (1,4)-N-acetylglucosaminyl transferase III to increase the percentage of non-core-fuccosylated proteins (US Patent 6,602,684).
A high content of glycoproteins without core fucose can also be achieved in mouse cells. However, these proteins contain the disadvantageous features typical for mouse proteins. A specific hybrid cell of a human a and a mouse with the right chromosomal composition cell can combine the advantageous features of both. Cell lines derived from H-CB-P1 such as PBG04 are such cell lines.

The present invention is further explained by the following examples.

The cell line H-CB-P1 was deposited at the "Zentralinstitut für Molekularbiologie, Akademie der Wissenschaften der DDR", Robert-Rössle-Str. 10, Berlin Buch, DDR-1115 as ZIM-0517 on March 16, 1990 and was transferred to the DSMZ, Deutsche Sammlung von Mikroorganismen und Zellkulturen GmbH, Maschroder Weg 13, 38124 Braunschweig, Germany, on December 12, 2000 and here given the depositary number DSM ACC2104. The PBG03 clone D3 (pVHCµCES hobFcblas) was renamed PBG04 and was deposited at the DMSZ as DSM ACC2577 on September 18, 2002.

### Examples

### Materials and Methods

### Materials:

### DNA Cloning Techniques

Isolation of Genomic DNA: Cells from a T25 cm² flask were trypsinized (see chapter "Trypsinisation" below), the resuspended cell pellet transferred into a 1.5 ml Eppendorf tube and 200 µl PBS added. The tube was centrifuged for 5 min at 13,200 rpm, the supernatant discarded and the pellet re-suspended in 2 ml of solution A. After transfer of the suspension into a Falcon tube (15 ml), 133 µl 10% SDS and 333 µl protease K were added. Either a 3 h incubation at 55°C, or an over night incubation at room temperature followed. The suspension was mixed with 607 µl 6 M NaCl and vortexed for 15 s before centrifuging it (4300 rpm, 4°C, 20 min). The supernatant was transferred into a Falcon tube (15 ml) and mixed with 2.5 ml 100% Ethanol. A threadlike DNA precipitate formed at the interface and was removed with a pipette tip and re-suspended in ½ TE buffer. The DNA was allowed to completely dissolve at 56°C before it was stored at 4°C.

PCR: The PCR method was used to isolate genomic DNA sequences (preparative PCR) or to detect certain DNA sequences (analytical PCR).

Preparative PCR: Preparative PCR reactions (50 µl) were set up with the Expand High Fidelity PCR kit (Roche) according to the manufacturer's instruction (20-30 ng of template, 5 µl of 15 mM Mg Cl₂ buffer (10x), 5 µl dNTP mix, 0.5 µl of each primer (30 nM), 0.5 µl polymerase and filled to 50 µl with water). The PCR products were purified using a QIAquick PCR purification kit (QIAGEN).

Analytical PCR: Analytical PCR reactions (10 µl) were prepared with Taq polymerase kit (QIAGEN) following the manufacturer's instruction (10 ng of template, 1 µl 10x buffer, 0.5 µl dNTP mix, 0.1 µl of each primer (30 pM), 0.1 µl Taq polymerase and filled to 10 µl with water).

The PCR cycling program varied for each product according to the annealing temperature of the primer (see Table 2) and the length of the expected PCR products (determined elongation time and temperatur; see Table 1).

**Table 1: Length of the amplified fragment determines elongation time**

| parameter | length, time temperature | length, time temperature | length, time temperature | length, time temperature | length, time temperature |
|---|---|---|---|---|---|
| length of fragment | < 750 bp | 1.5 kb | <3 kb | > 3 kb | 6 kb |
| elongation time | 45 s | 1 min | 2 min | 3 min + 15 s after each step | 4 min + 15 s after each step |
| temperature | 72°C | 72°C | 72°C | 68°C | 68°C |

**Table 2: Annealing temperature for primers**

| Primer/SEQ ID NO: | Sequence (annealing temperature [°C]) | Producer |
|---|---|---|
| VHpromF/1 | **ATACTAGTCGGCCG**CAGGCACATCCACAGTCAC (55) | GIBCO BRL |
| VHpromR/2 | **TCCCGGGTATCGA**TGGAGCTCTCAGGGGATTC (55) | GIBCO BRL |
| CµintV/3 | **CATCGA**TCCGCTACTACTACTACATGG (55) | GIBCO BRL |
| CµintR/4 | CGGCCACGCTGCTCGTAT (55) | GIBCO BRL |
| CµMitteR/5 | AGCTCACCTGGTGCAACT (54) | GIBCO BRL |
| CµMitteF/6 | GACCTAAGCTGACCTAGAC (54) | GIBCO BRL |
| V5/7 | TCCCTC-CAAAAGCTGTAG (52) | TIB |
| V6/8 | ATGGCGGTAATGTTGGAC (52) | TIB |
| V7/9 | CACAAGAATCCGCACAGG (54) | TIB |
| EBVtestR/10 | CCTGATATTGCAGGTAGG (52) | GIBCO BRL |
| EBVtestF/11 | TACCGACGAAGGAACTTG (52) | GIBCO BRL |

In bold: restriction sites

Amplification, isolation and quantification of plasmid DNA: *E. coli* transformants were grown in a 1 ml, 30 ml or 100 ml culture and plasmid DNA isolated using Mini-, Midi- or Maxi- plasmid purification kits (QIAGEN), respectively. The instruction of the manufacturer were followed. The DNA concentration was determined via spectroscopy, measuring the absorbance at 260 and 280 nm.

Restriction Enzyme Digestion: Plasmid DNA was digested with 1 unit of the appropriate restriction enzyme for 1 µg of DNA, using the buffer and temperature recommended by the supplier (see Table 3). If the analysis required the use of two or more restriction enzymes, the reactions were carried out simultaneous digestion if possible. Otherwise, sequential single digestions were performed with an intervening column purification step (QIAGEN) of the reaction mix.

**Table 3: Used Restriction Enzymes**

| Enzyme | Conc. | Temp. | Buffer | Inact. | Producer | Art. No. |
|---|---|---|---|---|---|---|
| BamHI | 20 U/µl | 37°C | 2^{a} | 65°C | BioLabs | R0136L |
| BgIII | 40 U/µl | 37°C | M | 65°C | Boehringer | 1175068 |
| BsaBI | 10 //µl | 60°C | 2 | 80°C | BioLabs | R0556S |
| BsiWI | 10 U/µl | 55°C | 3 | 80°C | BioLabs | R0136L |
| Bst11071 | 5 U/µl | 37°C | 3 + BSA | 80°C | BioLabs | R0553S |
| BstBI | 20 U/µl | 65°C | 4 + BSA | 80°C | BioLabs | R0519S |
| ClaI | 10 U/µl | 37°C | H | 65°C | Roche | 404217 |
| DraIII | 20 U/µl | 37°C | 3 + BSA | 65°C | BioLabs | R0510L |
| EagI | 10 U/µl | 37°C | 3 + BSA | 65°C | BioLabs | R0505S |
| EcoRI | 40 U/µl | 37 °C | H | 65°C | Roche | 200310 |
| HincII | 10 U/µl | 37°C | 3 + BSA | 65°C | BioLabs | R0103S |
| HindIII | 40 U/µl | 37°C | B | 5°C | Roche | 798983 |
| KpnI | 10 U/µl | 37°C | L | 65°C | Roche | 899186 |
| Mfel | 10 U/µl | 37°C | 4 | 65°C | BioLabs | R0589S |
| NotI | 10 U/µl | 37°C | 3 + BSA | 65°C | BioLabs | R0189L |
| PmeI | 10 U/µl | 37°C | 4 + BSA | 65°C | BioLabs | R0560L |
| PstI | 10 U/µl | 37°C | H | 80°C | Roche | 621633 |
| PvuII | 5 U/µl | 37°C | 3 + BSA | 65°C | BioLabs | R0150S |
| SacII | 20 U/µl | 37°C | 4 + BSA | 65°C | BioLabs | R0157S |
| SalI | 10 U/µl | 37°C | H | 65°C | Boehringer | 567663 |
| ScaI | 10 U/µl | 37°C | H | 80°C | Roche | 775266 |
| SmaI | 20 U/µl | 37°C | 4 + BSA | 65°C | BioLabs | R0141S |
| SpeI | 10 U/µl | 37°C | 2 + BSA | 65°C | BioLabs | R0133L |
| SspI | 5 U/µl | 37°C | 2 + BSA | 65 | BioLabs | R0182L |
| StyI | 10 U/µl | 37°C | H | 65°C | Roche | 85111023 |
| Swal | 10 U/µl | 25°C | 3 + BSA | 65°C | BioLabs | R0604L |
| XbaI | 10 U/µl | 37°C | H | 65°C | Boehringer | 674265 |

| | | | | | | |
|---|---|---|---|---|---|---|
| a. specific buffer | | | | | | |

End repair of DNA with 5' protruding termini: To "blunt" 5' overhangs such as those produced by EcoRI, the digested DNA was treated with the Klenow fragment (Roche) according to the manufacturer's instruction. The endfilling reaction was stopped by a heat inactivation step (65°C, 20 min) and the DNA ethanol precipitated or directly subjected to gel purification.

Dephosphorylation of vector DNA: To prevent self-ligation of linearised vector DNA (see passage "Ligation" below) with compatible ends, DNA was dephosphorylated using alkaline phosphatase (AP) (Roche) according to the manufacturer's instruction. The AP was heat inactivated (65°C, 15 min) and the DNA gel purified (for electrophoresis see passage "Agarose Gel Electrophoresis" below) prior to use in a ligation reaction.

TOPO Cloning: PCR amplification products were cloned into TOPO vectors from Invitrogen. According to the instruction of the TOPO cloning kit, the purified PCR product (0.25-2 µl) was mixed with the salt solution (0.5 µl), water added to reach a volume of 2.5 µl and then the TOPO vector (0.5 µl) added. Following a 30 min incubation at room temperature, the reaction tube was transferred onto ice, 2 µl of the reaction added to "one shot chemically competent E. coli" and the cells incubated for 30 min on ice. The cells were heat-shocked (42°C, 30 s), immediately transferred back onto ice and 250 µl of room temperature SOC medium added. The transformation reaction was incubated for 1 h at 37°C with shaking (300 rpm) before the mixture was plated on LB plates containing either Kanamycin or ampicillin. The plates incubated of overnight at 37°C.

Ligation: All ligation reactions were carried out in 10 µl volumes with 0.1-1 µg of dephosphorylated vector and an excess of insert. The reaction contained 2 µl T4 ligation buffer (Gibco BRL) and 1 µl T4 ligase (Roche), and were incubated for two hours at 16°C or overnight at 4°C. The ligation reaction was transformed into bacteria.
To reduce the level of unwanted non-recombinants, ligations could be post-digested with a suitable restriction enzyme if there was a unique restriction site in the self-ligated vector. Following the digestion, the ligation reaction was ethanol precipitated in the presence of acrylamide (centrifugation at 4°C, 14,000 rpm, 15 min) before the re-dissolved DNA was used again in a transformation reaction

Transformation of competent bacteria: Competent *E*. *coli* XL2 (stored at -70°C) were thawed on ice, mixed with either the ligation reaction (also kept on ice, see passage "Ligation" above) or with 1-100 ng plasmid DNA (re-transformation) and incubated on ice for 20 min. Subsequently the transformation reaction was heat-shocked (30-60 s, 42°C), the tube returned onto ice and 205 µl SOC medium (free of any antibiotic) added and the reaction incubated shaking (300 rpm) at 37°C for 45 min. The transformation reaction was plated on LB plates containing an antibiotic (either kanamycin (40-60 µg/ml) or ampicillin (50-100 µg/ml)) and incubated overnight at 37°C. The bacterial colonies were counted and the efficiency of the transformation reaction calculated.

Agarose Gel Electrophoresis: DNA fragments were separated according to their length on 0.7-1.5% agarose gels. The agarose was dissolved in 1xTAE buffer and 2 µl ethidiumbromide/100 ml agarose added. When the agarose had dissolved, it was poured into a tray and allowed to set. The DNA sample was mixed with the loading buffer Orange G, loaded onto a horizontal gel and run at 40-90 V with 1xTAE as running buffer. The DNA/ethidium bromide complexes were visualized under UV light.

Gel Purification of DNA Fragments: The DNA was separated on an agarose gel (40-80 V) and DNA bands (visualised under UV light) of interest excised with a scalpel. Using a QIAquick gel extraction kit (QIAGEN), the DNA was extracted from the agarose block according to the manufacturer's instructions.

### Cell culture

Trypsinisation: Adhesive cells were harvested using trypsin. First the culture medium was removed and the cell monolayer washed with citrate buffer (pre-warmed to 37°C). A small amount of trypsin was added directly to the cell monolayer and incubated for 3-5 min at 37°C. Trypsinisation was stopped by addition of PBG 1.0 medium supplemented with 5% FCS (see Table 4). The cell suspension was transferred into a Falcon tube (50 ml) and centrifuged for 10 min (800 rpm, 30°C). The cell pellet was re-suspended in fresh medium and the cells used for electroporation or for further passaging.

**Table 4: Volumes of citrate, trypsin and PBG 1.0 supplemented with 5% FCS used for the trypsinisation of the cells growing in different flasks**

| Tissue culture flask | Citrate buffer (ml) | Trypsin (ml) | PBG 1.0/5%FCS (ml) |
|---|---|---|---|
| T25 | 1.0 | 0.5 | 4.5 |
| T85 | 2.0 | 1.0 | 9.0 |
| T180 | 5.0 | 2.0 | 8.0 |

### Counting of Cells

After the cells had been trypsinized they were counted in a Neubauer chamber (haematocytometer). A small volume of the cell suspension was introduced into the chamber and the chamber placed under a microscope. Only cells within one of the four squares of the chamber were counted, the cell number multiplied with the factor 10⁴ to obtain the number of cells per ml. To differentiate between vital and dead cells, the cells were stained with trypan blue prior to counting. Dead cells appeared blue whereas vital cells did not take up the dye.

### Transformation

Electroporation of H-CB-P1 Cells: In a standard electroporation reaction 10 µg of linearised plasmid DNA were used. The culture medium was removed and the H-CB-P1 monolayer washed with citrate buffer and trypsinised (see passage "Trypsinisation" above). The cell pellet was re-suspended in Opti-MEM (pre-warmed to 37°C) to obtain 3 x 10⁶ cells per ml. A volume of 700 µl of the cell suspension was transferred into the electroporation cuvette (peqLab; EQUBIO 4 mm) and the linearised DNA (10 µg) added. The cells were electroporated at 250 V, 1500 µF and immediately afterwards transferred into T75 bottles containing pre-warmed PBG 1.0 medium supplemented with 5% FCS, and incubated at 37°C and 5% CO₂.

### Selection

Selection of H-CB-P1 Cells: The electroporated H-CB-P1 cells were cultured for two days at 37°C and 5% CO₂. On day 2, the culture medium was removed and non-adhesive cells harvested by centrifugation of the culture medium. 1 ml of the culture medium (supernatant) was frozen for later examination of transient expression. The trypsinized cell monolayer and the cells harvested from the culture medium by centrifugation were combined and pelleted. Cells were re-suspended in PBG 1.0 medium supplemented with 5% FCS to obtain dilutions of 1x10⁶, 1x10⁵ and 1x10⁴ cells/ml. Each dilution was supplemented with 5 µg/ml or 10 µg/ml blasticidin or with 200 µg/ml or 400 µg/ml hygromycin. The selection medium was changed on days 4, 7 and 10, and the growth of the H-CB-P1 clones controlled via microscopy. Between days 8 and 10 vital clones were visible by eye. On day 13 clones were harvested by trypsinisation, the cell pellet suspended in PBG 1.0 medium so that when cells were seeded into 96 well plates, a well contained either 5 cell or 1 cell. The selection pressure (either 5 or 10 µg/ml blasticidin or 200 or 400 µg/ml hygromycin) was maintained throughout. On day 10 the cells were immuno-stained to differentiate between positive and negative cell clones. The cell culture medium was removed and replaced with standard medium supplemented with a fluorecently labeled antibody (2 µg/ml) recognizing the recombinant protein produced by the cells. The antibody suspension was left for 4 h on the cell monolayer before it was replaced with OptiMem 1 supplemented with 5% FCS. The cell monolayers were examined under a fluorescent microscope. When Texas red conjugated antibodies were used, the microscopy was done with a UV filter (WG) that is transmissible for 470-480 nm spectra. The excited Texas red labeled antibodies emit light in the spectra of 590 nm. A UV filter (WU) transmissible for spectra of 330-355 nm was used for visualization of antibodies conjugated to AMCA. Emission of excited AMCA occurred in the blue spectra (420 nm). Only clones that were large and strongly fluorescent were considered. Was there only a single clone in one well, the cells were further expanded. Was there more than only one clone in a well, the individual clones (cells) were picked with a microcapillar and transferred into a new 96 well plate for further expansion (see the following passage "Mircopillary Picking").

Microcapillary Picking: The microcapillary picking device used a capillary attached to a movable arm that was controlled via a joystick. The microcapillar and the arm were inside the hood wheras the joystick was controlled from outside. When an interesting clone was identified via the immunostaining technique (described in section "Selection of H-CB-P1" above), the microcapillary was placed over the clone, negative pressure induced within the capillary through a vacuum pump and the cell pile of interest sucked into the microcapillary. The arm was moved over the fresh well of a 96 well plate and the cells therein ejected.

Cryoconservation: For long term storage cells trypsinized cells were re-suspended at 1x10⁶ to 1x10⁷ cells/ml. The cells were pelleted by centrifugation (700 rpm, 10 min) and the supernatant removed. The cells re-suspended in cold pre-conditioned medium (900 µl),a cryo-vial filled with 180 µl DMSO and 720 µl FCS, and the 900 µl cell suspension transferred into the DMSO/FCS solution. The cryo-vial was stored for 24 h in a special freezing container to freeze the cells gently. For long term storage the cryo-vials were transferred into liquid nitrogen tanks (storage at -196°C).

### Detection of Protein Products

EC-Western-Blot (Enhanced Chemiluminscence): For detection of hobFC antibodies 20 µl of cell culture supernatant were mixed with 10 µl 5% SDS and incubated for 2 min at 97°C. Was the cell culture medium expected to contain IgM antibodies, the culture medium was not treated. A membrane (Amersham-Pharmacia; Hybond-P) was first rinsed in methanol (1 min), washed three times in water (1 min), and then soaked in plot transfer buffer before placing it on a piece of 3 MM paper also soaked with plot transfer buffer. 5 µl of the pre-treated (hobFC antibodies) or 5 µl of the untreated (IgM antibodies) culture medium were spotted onto the membrane and incubated for 1 min. The membrane was placed in blocking buffer, incubated for half an hour under shaking and three-times washed for 5 min in T-PBS. The blocked membrane was placed in a detecting antibody solution (for IgG 1:2000 and for IgM 1:5000) and incubated for 2 h. Three more washes, of 2, 5 and 10 min in T-PBS buffer followed. The membrane was placed in developer (Amersham-Pharmacia, ECL) and incubated for 1 min. Finally it was drained and placed onto 3 MM paper and wrapped in cellophane to prevent drying. The light emission was observed in a dark room.

### Example 1: Preparation of a targeting vector specific for the IgM region of H-CB-P1 cells

The recombinant gene was to be inserted into the IgM sequence region because it is well-known that antibodies are highly expressed and secreted proteins. The final targeting vector hence required sequences that had 100% homology to the targeted genomic IgM sequences. For the basic targeting vector pVHCµ the VH region of 2 kb and the Cµ intron region of 7.4 kb in length were chosen (see Fig. 3). Both regions were isolated as PCR fragments using polymerases with proof-reading activity (proofstart polymerase (Qiagen)), subcloned into a pCR 4BluntTOPO vector (Invitrogen) and finally combined in one vector named pVHCµ (see Fig. 3).

Preparation of plasmids pVHCµCESHhobFc and pVHCµHhobFc: The basic targeting vector pVHCµ does not have an endogenous cassette yet. The endogenous cassette containing the CE promoter, the place holder gene hobFc, three FRT recombination sites as well as a hygromycin resistance gene and an ATG deleted neomycin gene was isolated from the vector pCESHhobFc as a BstDI-SwaI fragment. The fragment was then endfilled with Klenow polymerase and ligated into the basic targeting vector pVHCµ that had been digested with PmeI and dephosphorylated. The resulting targeting vector was called pVHCµCESHhobFc.

A second vector pVHCµHhobFc that lacked the CES promoter construct but contained all the other parts of the endogenous cassette was also constructed. A BstBI-Bstl107I fragment was isolated from pCESHhobC and endfilled. The Bstl107I restriction site in pCESHhobFc is immediately upstream of the frt wt site followed by the hobFc gene and thus the isolated Bstl107I-Bstb1 fragment lacks the promoter. The fragment was ligated into a pVHCµ vector previously digested with PmeI and the resulting vector was pVHCµHhobFc. In both vectors pVHCµCSHHobFc and pVHCµHhobFc the active resistance marker gene of the endogenous cassette was the hygromycin resistance gene. An alternative set of vectors that contained a blasticidin gene instead of the hygromycin gene was also created.

Construction of pVHCµCSHhobFcblas and pVHCµhobFcblas: To create targeting vectors with the blasticidin gene as marker gene in the endogenous cassette, the vector pcDNATRD was used as donor for the blasticidin gene. The first step involved the exchange of the hygromycin gene with the blasticidin gene. To this end the blasticidin gene was isolated from pcDNATRD as an EcoRI-SalI fragment. The endogenous cassette vector pcESHhobFc was also opened with EcoRI-SalI and thereby the hygromycin gene was removed as well as part of the ATG-deleted neomycin gene. The fragment containing the blastidin gene was ligated into the opened pCESHhobFc and the resulting vector named pCEShobFcblasdeleted.

The second step was the re-insertion of the coincidentally removed ATG-deleted neomycin gene. For that a fragment encompassing the ATG-deleted neomycin gene was isolated from pCESHhobFc and inserted into the opened vector pCEShobFcblasdeleted. The resulting vector was named pCEShobFcblas. This vector now served as donor vector for the blasticidin gene for plasmids pVHCµCSHhobFc and pVHCµHhobFc. A BamHI-SalI fragment was isolated from pCESHhobFcblas and inserted into a BamHI-SalI opened vector pVHCµCESHhobFc, giving rise to pVHCµCEShobFcblas. To create the control vector pVHCµhobFcblas lacking the CES promoter, the vector pVHCµHhobFc was also digested with BamHI-SalI and again the BamHI-SalI fragment isolated from pCEShobFcblas ligated into it.

### Example 2: Selection of hobFc clones

Electroporation: H-CB-P1 cells were electroporated with plasmids pVHCµCshobFcblas, pVHCµhobFcblas, pVHCµHhobFc and pCShobFcblas. In order to determine the transfection efficiency, cells were transfected with plasmid pGFPN1VA and as mock control, cells were electroporated with a water sample. The transfection efficiency was found to be at approximately 20%. On day 2 post-electroporation depending on the transfected plasmid either hygromycin or blasticidin was added to the culture medium. When mock-transfected cells were all dead, cells from the other transfection reactions were harvested and re-seeded at a density of either 1 cell or 5 cells per well into a 96 well plate. Cells were continued to be cultured with medium supplemented with the appropriate antibiotic.

To optimise the selection conditions cells were seeded at 10⁴, 10⁵ or 10⁶ cells/20 ml into T75 flasks two days post-electroporation. The medium was supplemented with either 5 or at 10 µg/m blasticidin,or 200 or 400 µg/ml hygromycin. On day 14 the number of clones per cm² was determined. The highest number of clones was obtained in flasks seeded with at 1 x 10⁶ cells that had been transfected with the plasmids lacking the CES promoter (pVHCµhobFcblas). Three times fewer clones were obtained when cells had been transfected with plasmids carrying the CES promoter. Furthermore these clones did grow less well as those without the CES promoter.

The effect-antibiotics have on the selection of protein producing clones: Following the expansion of the clones in T75 flasks, the clones were trypsinized and re-seeded into 96 well plates at 5 cells/well. The culture medium contained either 5 or 10 µg/ml blasticidin or 200 or 400 µg/ml hygromycin. On day 10 post-seeding, cells were stained with Anti IgG antibodies conjugated with Texas red and AMCA labeled antibodies against IgM. The results obtained with cells cultured with blasticidin showed that with the higher antibiotic concentration far fewer positive clones were obtained. When 10 µg/ml blasticidin were used, 100% more hobFC negative clones were observed compared to 5 µg/ml. However, only the first three rows of the 96 well plate containing cells cultured with 10 µg/ml blasticidin were counted and the result for the entire plate was extrapolated wheras all rows of the 96 well plate containing cells cultured with 5 µg/ml blasticidin where counted.

Advantage of selection with fluorescently labeled antibodies: Using the immunofluorescent staining technique large numbers of clones can be screened rapidly. The results obtained with the direct immuno-technique were a first indication for correct integration of the targeting sequence into the genomic DNA. Non-expressing clones were easily detected with this technique. The immunofluorescent staining method is technically easy, lower concentrations of antibodies are required than with the methyl-cellulose staining technique, and the proliferation of cells is not impaired. Without any noticeable damages, cells could be immuno-stained before subsequent trypsinization or microcapillary picking to transfer cells into new culture vessels.

### Example 3: Detection of homologous insertion into the IgM locus

For the design of the targeting vector the rearranged immunoglobulin heavy chain locus was assembled based on the cDNA sequence of the antibody and human genome sequence information. To ensure that the production of IgM was disrupted by the targeting approach, the complete leader sequence including the ATG, the V, D and J genes were omitted from the targeting vector and deleted from the genome via a single homologous recombination event. Hence the replacement type targeting vector contained isogenic sequences from the IgM locus to allow the directed cross over as well as the hobFc gene, blasticidin and ATG deleted neomycin resistance genes. Since only one rearranged active IgM locus on chromosome 14 is present in the starting cell line H-CB-P1, the homologous recombination event completely abolishes IgM expression which is detected by fluorescent antibody staining and supernatant immunoblotting using an anti-IgM antibody.

Western Blot (Dot Blot) for IgM and IgG: The dot blot technique was used to verify the results obtained with the direct immuno-staining technique. The supernatant (medium) of these clones was examined for presence of IgM and IgG. If supernatant was found to be IgM negative as well as IgG positive, it was concluded that a homologous recombination event had taken place.

PCR for detection of integrated targeting sequences: To verify that the targeting cassette had become integrated at the IgM locus PCR reactions were set up with the forward primer V5 (SEQ ID NO:7) and reverse primers V6 (SEQ ID NO:8) or V7 (SEQ ID NO:9), and genomic DNA isolated from cell clones as template. Primer V5 binds to the genomic V gene promoter sequences outside of the fragment Vhprompresent in the targeting vector, reverse primer V6 binds within the CES promoter sequences (and hence was only used for cells transfected with plasmids carrying the CES promoter) and primer V7 bind within the hobFc gene. Using the described primer combinations the occurrence of PCR products is strictly dependent on co-localisation of both primer binding sequences and hence homologous recombination. To increase the sensitivity of this PCR assay, first-round PCR products were used as templates in nested PCR reactions with primers VHpromF (SEQ ID NO:1) and VHpromR (SEQ ID NO:2). Finally nested PCR products were subjected to an enzyme restriction digest with HincII and DraI to confirm that obtained sequences were correct. Passing on all these assays, PBG03 clones H6 (pVHCµhobFcblas), D4 (pVHCµhobFcblas), E11 (pVHCµhobFcblas) D3 (pVHCµCEShobFcblas) and G8 (pVHCµhobfcblas) had integrated the targeting sequence correctly. Clone D3 (pVHCµCEShobFcblas) was renamed PBG04 and deposited at the German Collection of Microorganism and Cell Cultures (DSMZ).

### Example 4: Recombination to generate a target cell clone

Clone pVHCµCEShobFcblas D3 (PBG04) was transfected with vector 2 comprising a second functional sequence (frtwt, GFP ORF and polyadenylation signal, minimal promoter followed by ATG and frt5) and plasmid pflp comprising a functional expression unit for flp recombinase using the transfection reagent effectene (Qiagen). Vector 2 does not contain a promoter driving the GFP expression unit. The functionalised cell (PBG04) is sensitive to Geneticin selection from 200 µg/ml. Vector 2 misses a neomycin resistance gene sequence which could confer resistance to Geneticin. As expected no green fluorescence was detectable 1-4 days after transfection. After two weeks of selection with Geneticin individual stable clones strongly expressing GFP were detectable. GFP expression depends on integration in direct proximity to a functional promoter. Geneticin resistance is dependent on reconstitution of the neo resistance gene present in the cell line by the ATG from vector 2. We conclude that in all cases vector 2 has replaced sequences between the frtw and frt F5 sites and functionally linked the CES promoter and GFP as well as the ATG deleted neomycin gene with the ATG.

### Example 5: Studies of the glycosylation pattern of Leptin Fc

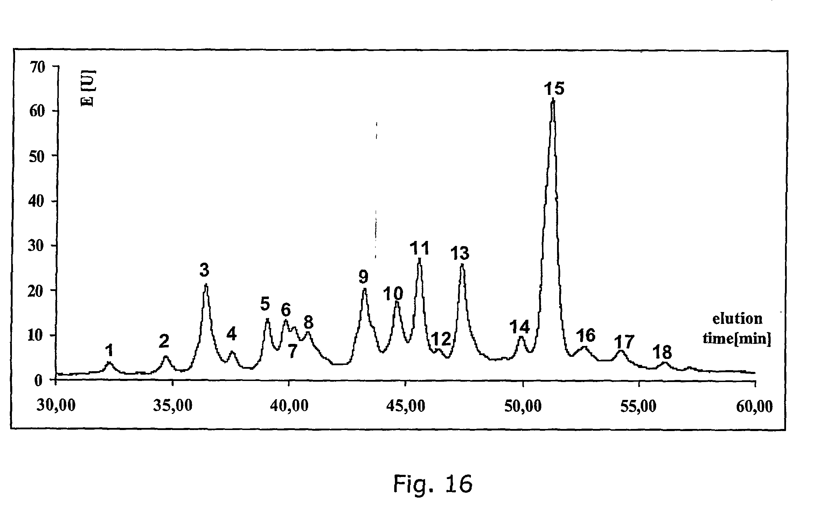

Leptin Fc from PB604 was generated in roller bottle culture and purified by a generic process including affinity chromatography, gel filtration and membrane filtration. The protein was digested with trypsin and the resulting peptides were deglycosylated by PNGase F digestion. The glycans were labeled with 2-aminobenzamide and separated by HPLC on a Phenomenex Hypersil APS-2-column (Fig.16). MALDI-TOF-MS (BRUKER BIFLEX^{™}) was used with the desialylated, labelled samples to further characterize the respective fractions shown in Table 5.

The single N-glycosylation site on Fc carries complex oligosaccharide structures which are sialylated at 37% , a rate close to average sialylation on antibodies in human blood. Sialic acids were further characterized by sialidase treatment, DMB labelling and separation on a Bischoff Hypersil-ODS-column and compared with the Sialic Acid Reference Panel (Oxford GlycoSciences). Typically, N-acetylneuraminic acid was found. Only 2% were represented by N-glycolylneuraminic acid, the dominating form in mouse myeloma cells; which was shown to be immunogenic (Noguchi, A. et al., J. Biochem, 17(1):p. 59-62 (1995)). Alpha 1,3 Gal structures, which are not made in human cells and are known to increase clearence via preexisting antibodies, were only found in 1.3% of the glycans. The above findings are summarized in Table 6.

**Tab. 5: Identified oligosacharides as the result of Results of MALDI-TOF-MS analysis of fractions from the Phenomenex Hypersil APS-2-column**

| *Peak* | *Area (%)* | *Monoisotopic mass (m*/*z)* | *Proposed structure* |
|---|---|---|---|
| 1 | 1,2 | 1256,9 | Man3HexNAc1 |
| 2 | 1,4 | 1402,9 | Man3HexNAc1Fuc |
| 3 | 8,0 | 1378,4 Main | High Man5 |
| | | 1377,9 | High Man5 |
| 4 | 1,6 | 1419,0 | Man3HexNAc1Hex1 |
| 5 | 4,3 | 1606,5 | Man3HexNAc2Fuc |
| 6 | 3,1 | 1565,5 | Man3HexNAc1Hex1Fuc |
| 7 | 2,7 | 1540,7 Trace | HighMan6 |
| | | 1581,7 Main | Man5HexNAc |
| | | 1540,5 | HighMan6 |
| 8 | 4,7 | 1581,5 Main | Man5HexNAc |
| | | 1622,7 | Man3HexNAc2Hex1 |
| 9 | 9,3 | 1768,8 | Man3HexNAc2Hex1Fuc |
| | | 1743,8 Main | Man5HexNAc1Hex1 |
| 10 | 6,6 | 1784,9 Trace | Man3HexNAc2Hex2 |
| 11 | 8,7 | 1784,7 | Man3HexNAc2Hex2 |
| | 1,6 | 1784,5 | Man3HexNAc2Hex2 |
| 12 | | 1889,7 | Man5HexNAc1Hex1Fuc |
| | | 1930,7 Trace | Man3HexNAc2Hex2Fuc |
| 13 | 10,9 | 1930,8 | Man3HexNAc2Hex2Fuc |
| 14 | 3,0 | 1664,5 | Bi - 2AB |
| | | 1946,9 Trace | Man3HexNAc2Hex3 (Bi + Gal*) |
| | | 2093,0 | Man3HexNAc2Hex3Fuc (Bi+Fuc+Gal*) |
| 15 | 25,7 | 2150,0 | Man3HexNAc3Hex3 |
| 16 | 3,2 | N.D. | |
| 17 | 2,8 | N.D. | |
| 18 | 0,9 | 2031,2 | Tri - 2AB |
| | | 2514,2 | Man3HexNAc4Hex4 |

**Tab. 6: Summary of specific features of N linked Oligosacharides from proteins isolated from human blood, hamster CHO, mouse NS0 cells or the heterohybridoma PBG-04**

| Feature | impact | PBG-04 | CHO | NS0 | human |
|---|---|---|---|---|---|
| Sialylation | 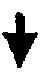 proteol. Sens./Clearence | 37% | variable | variable | 35-40 |
| N acetyl- | wanted | 98% | high | low | 100% |
| N glycolyl- | immunogenic | 2% | low | high (>50%) | no |
| 2-6 linkage | unknown | no | no | no | variable |
| | | | | | |
| 1-3 alpha gal. | Preexist. Ab: clearence | 1,3% | variable | high | no |
| | | | | | |
| Bisecting GlcNAc | ? → 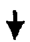 core fucosylation | no | no | no | 10% |
| No core fucose | 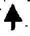 ADCC, Fcγ-binding | 60% | 5% | 10-50% | 5% |
| | | | | | |
| G0-structures | 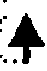 Dimerisation, G2→ 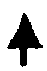 CDC | 4,3% | variable | variable | low |

### Example 6: Preparation of a targeting vector for the light chain lambda locus of PBG04

The structure of the rearranged lambda chain locus was identified by alignment of the known cDNA of the lambda gene with human genomic sequences. The gene consists of a variable gene, J and H segment already joined together. V3-19 was identified as the variable gene.
This approach was not suitable to identify the constant gene because the locus contains 100% identical gene copies. A PCR based on primers in the known leader sequence and in the unique intervening sequences between constant region genes. Primers V81, V83 (SEQ ID NOs:13 and 14, respectively) gave a correctly sized PCR product which showed the expected restriction pattern and therefore allowed to identify J2 and H2 as the genes constituting the rearranged lambda gene of H-CB-P1 (SEQ ID NO:21). Based on this information the sequence of the rearranged locus was proposed and a targeting vector was constructed.
A 5' flank upstream of the coding sequences of the variable gene V3-19 was created with Primers V89 and V94 (SEQ ID NOs:15 and 18, respectively) using Provestart Polymerase (Qiagen). A 4kb fragment was cloned in pPCR4blunttopo (Invitrogen). A 3 prime flank was amplified in two steps: overlapping PCR products were created using Primers V90 V91 and V115 V116 (SEQ ID NOs:16, 17, 19 and 20, respectively) and lined via a unique SphI site present in both fragments. The flanking sequences were cloned into a single vector PVLCL (SEQ ID NO:22).
To allow the insertion of genes independent from those in the heavy chain locus analogous but hetero-specific frt based replacement system was designed. It contains in the 5'3'direction a frt F3 site, the CMV EF1alpha hybrid promoter followed by the human alpha (1) antitrypsin gene, the hygromycin resistance marker, an wt frt site and an ATG deleted histidinol resistance marker. These elements were cloned into pVLCL to create pVLCLaathyg.
Since frt wt and F5 sites do not allow recombination, specific replacement vectors can exclusively target the heavy and light chain loci, providing a promoter and a start codon to the neomycin and histidinol resistance markers respectively. To increase selectivity, the replacement vectors contain start condons in different open reading frames relative to the frt site. As a result, incorporation of the vector into the wrong frt site does not result in resistance to the respective antibiotic.
PVLCLaathyg was transfected into PBG-04 using electroporation. Cells were seeded into a T75 flask and subjected to selection at 200µg/ml Hygromycin. For 3 weeks. Resulting clones were isolated by dilution cloning and clones resulting from homologous exchange were identified by the absence of an immunoflourescence signal using a fluorescence-labelled anti human-lambda-chain antibody. The resulting cell clones are analyzed for the expression of alpha 1 antitrypsin. These clones are suitable for the coexpression of two independent transgenes which have to be expressed at high level. Preferably these genes are the heavy and light chain genes of an antibody. Within a single exchange reaction using flp recombinase, heavy and light chain genes can be directed to their respective locations.

## Claims

1. An ex-vivo process for preparing a cell capable of stable high yield expression of a target gene product having an essentially human glycosylation pattern, which method comprises:
(a) selecting an immortalized human cell or human hybrid cell (starting cell) which is derived from B lymphocytes and is capable of stable high yield expression of an immunoglobulin (Ig) being non-essential to the starting cell;
(b) screening for the locus of the Ig gene within the genome of the starting cell;
(c1) replacing the gene coding for the Ig with a first functional DNA sequence containing one or more recombinase recognition sites (RRS) to obtain a functionalized precursor cell; and
(d) integrating a second functional DNA sequence containing a DNA sequence coding for the target gene product into the functionalized precursor cell obtained in step (c1) by use of a recombinase recognizing the RRSs incorporated with the first functional sequence, or
(c2) directly replacing the gene coding for the Ig with a functional DNA sequence containing a DNA sequence coding for the target gene product.

2. The process of claim 1, wherein
(i) the starting cell secretes the Ig, preferably in an amount of at least 0.3 fmol/cell/d of a polypeptide chain and more preferably in an amount of more than 1 fmol/cell/d; and/or
(ii) if the starting cell is a human hybrid cell, the Ig gene is a human gene.

3. The process of claim 1 or 2 wherein the starting cell is a human myeloma or hybridoma, or human hetero-hybridoma cell and most preferably is human-mouse hetero-hybridoma H-CB-P1 deposited as DSM ACC 2104.

4. The process according to any one of claims 1 to 3, wherein the integration of the functional DNA sequence(s) is effected at a rearranged Ig locus, preferably at a rearranged immunoglobulin H locus or λ locus of said starting cell.

5. The process according to any one of claims 1 to 4 wherein the locus of the Ig gene
(i) is known or is determined by a screening procedure comprising microarray expression analysis, 2D protein gel electrophoresis, quantitative PCR, RNAse protection, northern blot, ELISA, western blot and combinations thereof; and/or
(ii) is selected as to provide for an essentially human glycosylation pattern.

6. The process according to any one of claims 1 to 5, wherein the replacement of the Ig gene is effected
(i) by an one step replacement strategy, wherein the starting cell is contacted with a vector construct containing the first functional sequence, said first functional sequence replacing the gene coding for the Ig; or
(ii) in a two- or multi-step strategy, wherein the gene coding for the Ig gene is deleted or inactivated and subsequently contacted with a vector construct containing the first functional sequence, said first functional sequence being incorporated at the site of the deleted/inactivated Ig.

7. The process of claim 1 or 6, wherein the first functional DNA sequence
(i) comprises one or more RRS(s) selected from IoxP, frt, attL and attR sites of lambdoid phages, and recognition sites for resolvases or phage C31 integrase, preferably RRS(s) capable of unidirectional integration such as modified loxP sites and frt sites; and/or
(ii) further comprises functional sequences selected from marker sequences, secretion proteins, promoters, enhancers, splice signals, polyadenylation signals and IRES elements; and/or
(iii) is flanked in the vector by sequences that are homologous to the target gene or adjacent sequences.

8. The process of any one of claims 1 to 7, wherein
(i) the integration of the second functional DNA sequence is effected by delivering a recombinases recognising the RRS(s) present in the first functional sequence together with, shortly before or after delivery of the second functional sequence;
(ii) the integrase is selected from Cre, Flp, φC31 integrase and resolvase;
(iii) the target gene product is selected from enzymes, hormones, cytokines, receptors, antibodies, antibody domains and fusion proteins comprising the gene product mentioned before;
(iv) the second functional DNA sequence further comprises functional sequences selected from promoter sequences, marker sequences, splice donor and acceptor sequences, and recombinase recognition sequences differing from RRS of the first functional sequence.

9. The process of any one of claims 1 to 6, wherein the gene coding for the Ig is directly replaced with a functional DNA sequence containing a DNA sequence coding for the target gene product.

10. A process for preparing a functionalized cell comprising the steps (a) to (c1) as defined in claims 1 to 6.

11. A functionalized cell obtainable by the process of claim 10.

12. A cell capable of high yield expression of a target gene product obtainable by the process of claims 1 to 9.

13. The cell of claim 12, wherein the target gene product is an antibody, preferably the cell is PBG04 deposited as DMS ACC2577.

14. The cell of claims 11, 12 or 13, which is derived from H-CB-P1 deposited as DSM ACC2104.

15. The cell of claim 13 or 14 further having its light chain inactivated or replaced with a gene coding for the same or a different target gene product.

16. A method for high yield expression of a target gene product which comprises cultivating a cell as defined in any one of claims 12 to 15.

## Patentansprüche

1. Ex-vivo-Verfahren zur Herstellung einer Zelle, die zu einer stabilen Expression eines Zielgenprodukts, das ein im Wesentlichen humanes Glycosylierungsmuster aufweist, in hoher Ausbeute befähigt ist, wobei das Verfahren Folgendes umfasst:
(a) Auswählen einer immortalisierten humanen Zelle oder Human-Hybrid-Zelle (Ausgangszelle), die von B-Lymphocyten abgeleitet ist und zu einer stabilen Expression eines Immunglobulins (Ig), das für die Ausgangszelle nicht essentiell ist, in hoher Ausbeute befähigt ist;
(b) Suchen nach dem Locus des Ig-Gens innerhalb des Genoms der Ausgangszelle durch Screening;
(c1) Ersetzen des Gens, das für das Ig codiert, durch eine erste funktionelle DNA-Sequenz, die eine oder mehrere Rekombinase-Erkennungsstellen (RRS) enthält, wobei man eine funktionalisierte Vorläuferzelle erhält; und
(d) Integrieren einer zweiten funktionellen DNA-Sequenz, die eine DNA-Sequenz enthält, welche für das Zielgenprodukt codiert, in die in Schritt (c1) erhaltene funktionalisierte Vorläuferzelle durch Verwendung einer Rekombinase, die die in die erste funktionelle Sequenz eingebauten RRS-Stellen erkennt; oder
(c2) direktes Ersetzen des Gens, das für das Ig codiert, durch eine funktionelle DNA-Sequenz, die eine DNA-Sequenz enthält, welche für das Zielgenprodukt codiert.

2. Verfahren gemäß Anspruch 1, wobei:
(i) die Ausgangszelle das Ig sezerniert, und zwar vorzugsweise in einer Menge von wenigstens 0,3 fmol einer Polypeptidkette pro Zelle pro Tag und besonders bevorzugt in einer Menge von mehr als 1 fmol/Zelle/Tag; und/oder
(ii) wenn die Ausgangszelle eine Human-Hybrid-Zelle ist, das Ig-Gen ein humanes Gen ist.

3. Verfahren gemäß Anspruch 1 oder 2, wobei die Ausgangszelle eine humane Myelom- oder Hybridom- oder Human-Hetero-Hybridomzelle ist und es sich dabei am meisten bevorzugt um das Human-Maus-Hetero-Hybridom H-CB-P1 handelt, das als DSM ACC 2104 hinterlegt ist.

4. Verfahren gemäß einem der Ansprüche 1 bis 3, wobei die Integration der funktionellen DNA-Sequenz bzw. DNA-Sequenzen an einem umgelagerten Ig-Locus, vorzugsweise an einem umgelagerten Immunglobulin-H-Locus oder λ-Locus der Ausgangszelle, durchgeführt wird.

5. Verfahren gemäß einem der Ansprüche 1 bis 4, wobei der Locus des Ig-Gens:
(i) bekannt ist oder durch ein Screening-Verfahren bestimmt wird, das eine Mikroarray-Expressionsanalyse, 2D-Protein-Gel-Elektrophorese, quantitative PCR, RPA (RNase protection assay), Northern Blot, ELISA, Western Blot und Kombinationen davon umfasst; und/oder
(ii) so ausgewählt wird, dass man ein im Wesentlichen humanes Glycosylierungsmuster erhält.

6. Verfahren gemäß einem der Ansprüche 1 bis 5, wobei das Ersetzen des Ig-Gens durchgeführt wird:
(i) durch eine einstufige Ersatzstrategie, wobei die Ausgangszelle mit einem Vektorkonstrukt in Kontakt gebracht wird, das die erste funktionelle Sequenz enthält, wobei die erste funktionelle Sequenz das für das Ig codierende Gen ersetzt; oder
(ii) in einer zwei- oder mehrstufigen Strategie, wobei das für das Ig codierende Gen deletiert oder inaktiviert und die Ausgangszelle anschließend mit einem Vektorkonstrukt in Kontakt gebracht wird, das die erste funktionelle Sequenz enthält, wobei die erste funktionelle Sequenz an der Stelle des deletierten/inaktivierten Ig eingebaut wird.

7. Verfahren gemäß Anspruch 1 oder 6, wobei die erste funktionelle DNA-Sequenz:
(i) eine oder mehrere RRS-Stellen, die aus loxP-, frt-, attL- und attR-Stellen von lambdoiden Phagen ausgewählt sind, und Erkennungsstellen für Resolvasen oder Phagen-C31-Integrase, vorzugsweise RRS-Stellen, die zu einer unidirektionalen Integration befähigt sind, wie modifizierte loxP-Stellen und frt-Stellen, umfasst; und/oder
(ii) weiterhin funktionelle Sequenzen umfasst, die aus Markersequenzen, Sekretionsproteinen, Promotoren, Enhancern, Spleißsignalen, Polyadenylierungssignalen und IRES-Elementen ausgewählt sind; und/oder
(iii) im Vektor von Sequenzen flankiert ist, die zum Ziel-Gen oder benachbarten Sequenzen homolog sind.

8. Verfahren gemäß einem der Ansprüche 1 bis 7, wobei:
(i) die Integration der zweiten funktionellen DNA-Sequenz durchgeführt wird, indem man zusammen mit, kurz vor oder nach der Einschleusung der zweiten funktionellen Sequenz eine Rekombinase einschleust, die die in der ersten funktionellen Sequenz vorhandenen RRS-Stellen erkennt;
(ii) die Integrase aus Cre-, Flp-, φC31-Integrase und Resolvase ausgewählt ist;
(iii) das Zielgenprodukt aus Enzymen, Hormonen, Cytokinen, Rezeptoren, Antikörpern, Antikörperdomänen und Fusionsproteinen, die das oben genannte Genprodukt umfassen, ausgewählt ist;
(iv) die zweite funktionelle DNA-Sequenz weiterhin funktionelle Sequenzen umfasst, die aus Promotorsequenzen, Markersequenzen, Spleißdonor- und -akzeptorsequenzen sowie Rekombinase-Erkennungssequenzen, die von der RRS der ersten funktionellen Sequenz verschieden sind, ausgewählt sind.

9. Verfahren gemäß einem der Ansprüche 1 bis 6, wobei das für das Ig codierende Gen direkt durch eine funktionelle DNA-Sequenz ersetzt wird, die eine DNA-Sequenz enthält, welche für das Zielgenprodukt codiert.

10. Verfahren zur Herstellung einer funktionalisierten Zelle, das die Schritte (a) bis (c1) umfasst, wie sie in den Ansprüchen 1 bis 6 definiert sind.

11. Funktionalisierte Zelle, die nach dem Verfahren gemäß Anspruch 10 erhältlich ist.

12. Zelle, die zu einer Expression eines Zielgenprodukts, das nach dem Verfahren gemäß den Ansprüchen 1 bis 9 erhältlich ist, in hoher Ausbeute befähigt ist.

13. Zelle gemäß Anspruch 12, wobei das Zielgenprodukt ein Antikörper ist und es sich bei der Zelle vorzugsweise um PBG04 handelt, die als DSM ACC 2577 hinterlegt ist.

14. Zelle gemäß Anspruch 11, 12 oder 13, die von H-CB-P1 abgeleitet ist, das als DSM ACC 2104 hinterlegt ist.

15. Zelle gemäß Anspruch 13 oder 14, deren leichte Kette weiterhin inaktiviert oder durch ein Gen, das für dasselbe oder ein anderes Zielgenprodukt codiert, ersetzt ist.

16. Verfahren zur Expression eines Zielgenprodukts in hoher Ausbeute, umfassend das Kultivieren einer Zelle, wie sie in einem der Ansprüche 12 bis 15 definiert ist.

## Revendications

1. Procédé *ex vivo* pour la préparation d'une cellule susceptible d'une expression à haut rendement stable d'un produit d'un gène cible possédant un motif de glycosylation essentiellement humain, lequel procédé comprend :
(a) la sélection d'une cellule humaine immortalisée ou d'une cellule hybride humaine (cellule de départ) qui est dérivée de lymphocytes B et est susceptible d'une expression à haut rendement stable d'une immunoglobuline (Ig) étant non essentielle à la cellule de départ ;
(b) le criblage pour le locus du gène d'Ig à l'intérieur du génome de la cellule de départ ;
(c1) le remplacement du gène codant pour l'Ig par une première séquence d'ADN fonctionnelle contenant un ou plusieurs sites de reconnaissance de recombinase (RRS) pour obtenir une cellule précurseur fonctionnalisée ; et
(d) l'intégration d'une seconde séquence d'ADN fonctionnelle contenant une séquence d'ADN codant pour le produit du gène cible dans la cellule précurseur fonctionnalisée obtenue à l'étape (c1) par l'utilisation d'une recombinase reconnaissant les RRS incorporés avec la première séquence fonctionnelle ; ou
(c2) le remplacement direct du gène codant pour l'Ig par une séquence d'ADN fonctionnelle contenant une séquence d'ADN codant pour le produit du gène cible.

2. Procédé selon la revendication 1, dans lequel
(i) la cellule de départ sécrète l'Ig, de préférence dans une quantité d'au moins 0,3 fmol/cellule/d d'une chaîne polypeptidique et plus préférentiellement dans une quantité de plus de 1 fmol/cellule/d; et/ou
(ii) si la cellule de départ est une cellule hybride humaine, le gène d'Ig est un gène humain.

3. Procédé selon la revendication 1 ou 2 dans lequel la cellule de départ est un myélome ou un hybridome humain, ou une cellule d'hétérohybridome humain et le plus préférentiellement est l'hétérohybridome humain-murin H-CB-P1 déposé sous le numéro DSM ACC2104.

4. Procédé selon l'une quelconque des revendications 1 à 3, dans lequel l'intégration de la(des) séquence(s) d'ADN fonctionnelle(s) est effectué sur un locus d'Ig réarrangé, de préférence sur un locus H ou un locus λ d'immunoglobuline réarrangé de ladite cellule de départ.

5. Procédé selon l'une quelconque des revendications 1 à 4 dans lequel le locus du gène d'Ig
(i) est connu ou est déterminé par une procédure de criblage comprenant une analyse de l'expression sur puce à ADN, une électrophorèse sur gel protéique 2D, une PCR quantitative, une protection à la RNase, un transfert d'ARN, un ELISA, un transfert Western et des combinaisons de ceux-ci ; et/ou
(ii) est choisi de manière à fournir un motif de glycosylation essentiellement humain.

6. Procédé selon l'une quelconque des revendications 1 à 5, dans lequel le remplacement du gène d'Ig est effectué
(i) par une stratégie de remplacement en une étape, dans laquelle la cellule de départ est mise en contact avec une construction de vecteur contenant la première séquence fonctionnelle, ladite première séquence fonctionnelle remplaçant le gène codant pour l'Ig ; ou
(ii) dans une stratégie à deux ou de multiples étapes, dans laquelle le gène codant pour l'Ig est délété ou inactivé et ensuite mis en contact avec une construction de vecteur contenant la première séquence fonctionnelle, ladite première séquence fonctionnelle étant incorporée sur le site de l'Ig délétê/inactivé.

7. Procédé selon la revendication 1 ou 6, dans lequel la première séquence d'ADN fonctionnelle
(i) comprend un ou plusieurs RRS choisis parmi les sites loxP, frt, attL et attR de phages lambdoïdes, et des sites de reconnaissance pour des résolvases ou l'intégrase du phage C31, de préférence un(des) RRS susceptible(s) d'une intégration unidirectionnelle tel(s) que des sites loxP et des sites frt modifiés ; et/ou
(ii) comprend en outre des séquences fonctionnelles choisies parmi des séquences de marqueurs, des protéines de sécrétion, des promoteurs, des amplificateurs, des signaux d'épissage, des signaux de polyadénylation et des éléments IRES ; et/ou
(iii) est flanquée dans le vecteur par des séquences qui sont homologues du gène cible ou de séquences adjacentes.

8. Procédé selon l'une quelconque des revendications 1 à 7, dans lequel
(i) l'intégration de la seconde séquence d'ADN fonctionnelle est effectuée en délivrant une recombinase reconnaissant le(s) RRS présent(s) dans la première séquence fonctionnelle conjointement avec, juste avant ou après la délivrance de la seconde séquence fonctionnelle ;
(ii) l'intégrase est choisie parmi l'intégrase Cre, Fip, φC31 et une résolvase ;
(iii) le produit du gène cible est choisi parmi des enzymes, des hormones, des cytokines, des récepteurs, des anticorps, des domaines d'anticorps et des protéines de fusion comprenant le produit du gène mentionné précédemment ;
(iv) la seconde séquence d'ADN fonctionnelle comprend en outre des séquences fonctionnelles choisies parmi des séquences de promoteurs, des séquences de marqueurs, des séquences de donneurs et d'accepteurs d'épissage, et des séquences de reconnaissance de recombinase différents des RRS de la première séquence fonctionnelle.

9. Procédé selon l'une quelconque des revendications 1 à 6, dans lequel le gène codant pour l'Ig est directement remplacé par une séquence d'ADN fonctionnelle contenant une séquence d'ADN codant pour le produit du gène cible.

10. Procédé de préparation d'une cellule fonctionnalisée comprenant les étapes (a) à (c1) telles que définies dans les revendications 1 à 6.

11. Cellule fonctionnalisée pouvant être obtenue par le procédé selon la revendication 10.

12. Cellule susceptible d'une expression à haut rendement d'un produit d'un gène cible pouvant être obtenue par le procédé selon les revendications 1 à 9.

13. Cellule selon la revendication 12, dans laquelle le produit du gène cible est un anticorps, de préférence la cellule est le PBG04 déposé sous le numéro DSM ACC2577.

14. Cellule selon la revendication 11, 12 ou 13, qui est dérivée du H-CB-P1 déposé sous le numéro DSM ACC2104.

15. Cellule selon la revendication 13 ou 14 ayant en outre sa chaîne légère inactivée ou remplacée par un gène codant pour un produit de gène cible identique ou différent.

16. Procédé d'expression à haut rendement d'un produit d'un gène cible qui comprend la culture d'une cellule telle que définie dans l'une quelconque des revendications 12 à 15.


### SEQUENCE LISTING

<110> PROBIOGEN AG
<120> High Yield Heterologous Expression Cell Lines for Expression of Gene Products with Human Glycosylation Pattern
<130> 032353wo/JH
<140>
   <141>
<160> 22
<170> PatentIn Ver. 2.1
<210> 1
   <211> 33
   <212> DNA
   <213> Artificial Sequence
<220>
   <223> Description of Artificial Sequence: Primer VHpromF
<400> 1
   atactagtcg gccgcaggca catccacagt cac 33
<210> 2
   <211> 32
   <212> DNA
   <213> Artificial Sequence
<220>
   <223> Description of Artificial Sequence: Primer VHpromR
<400> 2
   tcccgggtat cgatggagct ctcaggggat tc 32
<210> 3
   <211> 27
   <212> DNA
   <213> Artificial Sequence
<220>
   <223> Description of Artificial Sequence: Primer C*µ*intV
<400> 3
   catcgatccg ctactactac tacatgg 27
<210> 4
   <211> 18
   <212> DNA
   <213> Artificial Sequence
<220>
   <223> Description of Artificial Sequence: Primer C*µ*intR
<400> 4
   cggccacgct gctcgtat 18
<210> 5
   <211> 18
   <212> DNA
   <213> Artificial Sequence
<220>
   <223> Description of Artificial Sequence: Primer
   C*µ*MitteR
<400> 5
   agctcacctg gtgcaact 18
<210> 6
   <211> 19
   <212> DNA
   <213> Artificial Sequence
<220>
   <223> Description of Artificial Sequence: primer
   C*µ*MitteF
<400> 6
   gacctaagct gacctagac 19
<210> 7
   <211> 18
   <212> DNA
   <213> Artificial Sequence
<220>
   <223> Description of Artificial Sequence: Primer V5
<400> 7
   tccctccaaa agctgtag 18
<210> 8
   <211> 18
   <212> DNA
   <213> Artificial Sequence
<220>
   <223> Description of Artificial Sequence: primer V6
<400> 8
   atggcggtaa tgttggac 18
<210> 9
   <211> 18
   <212> DNA
   <213> Artificial Sequence
<220>
   <223> Description of Artificial Sequence: primer V7
<400> 9
   cacaagaatc cgcacagg 18
<210> 10
   <211> 18
   <212> DNA
   <213> Artificial Sequence
<220>
   <223> Description of Artificial Sequence: Primer
   EBVtestR
<400> 10
   cctgatattg caggtagg 18
<210> 11
   <211> 18
   <212> DNA
   <213> Artificial Sequence
<220>
   <223> Description of Artificial Sequence: Primer
   EBVtestF
<400> 11
   taccgacgaa ggaacttg 18
<210> 12
   <211> 377
   <212> DNA
   <213> Artificial Sequence
<400> 12
   cagctggtgc agtctggggc tgaggtgaag aagcctgggg cctcagtgaa ggtctcctgc 60
   aaggcttctg gatacacctt caccggctcc tatatgcact gggtgcgaca ggcccctgga 120
   caaggccttg agtggatggg acggatcaat cctaacagtg gtggcacaaa ctatgcacag 180
   aaatttcagg gcagggtcac catgaccagg gacacgtcca tcagcacagc ctacatggag 240
   ctgagcaggc tgagatctga cgacacggcc gtgtattact gtgcgagaga caagctttcc 300
   cggtcagaag taccagctgg ccgctactac tactacatgg acgtctgggg caaagggacc 360
   acggtcaccg tctcctc 377
<210> 13
   <211> 18
   <212> DNA
   <213> Artificial Sequence
<220>
   <223> Description of Artificial Sequence: Primer V81
<400> 13
   agcttcggct caacacag 18
<210> 14
   <211> 18
   <212> DNA
   <213> Artificial Sequence
<220>
   <223> Description of Artificial Sequence: Primer V83
<400> 14
   gccttacctg cagagatg 18
<210> 15
   <211> 22
   <212> DNA
   <213> Artificial Sequence
<220>
   <223> Description of Artificial Sequence: Primer V89
<400> 15
   agtatacccc agaactctgc tt 22
<210> 16
   <211> 30
   <212> DNA
   <213> Artificial Sequence
<220>
   <223> Description of Artificial Sequence: Primer V90
<400> 16
   ggccgctgcg gccggaagat gaggctgact 30
<210> 17
   <211> 25
   <212> DNA
   <213> Artificial Sequence
<220>
   <223> Description of Artificial Sequence: Primer V91
<400> 17
   agcggccgct tgcaggacaa tatga 25
<210> 18
   <211> 18
   <212> DNA
   <213> Artificial.Sequence
<220>
   <223> Description of Artificial Sequence: Primer V94
<400> 18
   ttgcgtgaca ggctcagt 18
<210> 19
   <211> 18
   <212> DNA
   <213> Artificial Sequence
<220>
   <223> Description of Artificial Sequence: Primer V115
<400> 19
   atcacacggc acttctcg 18
<210> 20
   <211> 25
   <212> DNA
   <213> Artificial Sequence
<220>
   <223> Description of Artificial Sequence: Primer V116
<400> 20
   gagatatcgg cttctggagg acact 25
<210> 21
   <211> 14000
   <212> DNA
   <213> Artificial Sequence
<220>
   <223> Description of Artificial Sequence: Proposed sequence of the arranged light chain locus
<400> 21
<210> 22
   <211> 13685
   <212> DNA
   <213> Artificial Sequence
<220>
   <223> Description of Artificial Sequence: Vector pVLCL
<400> 22
   ctagtcctgc aggtttatcg gcttctggag gacactggga tcttgaattt gttttttgca 60
   ccaaccatca ctcaggccca ggattgaatt tattagaggg cattagtgtc gctaaggagt 120
   gaatttccac agatgggata ggtggtctct tctctgcaaa gttgaaccaa cccatggttc 180
   aacttttggt aacaccatgg ggtggtgcag agggtgctcc tctcctccca tgcagaggct 240
   gattgcccta gagtgcagtg ttctgggaac cttggtgggt ttcagcccca ccttctggag 300
   tagttctagt tggataggcc tggtcagtga ggcctggctg gtcccaatat gaagtgccag 360
   tcacatctgt tagggtaacc ttgggagcat ccctgcaccc ctgaatctgg cctcatgttg 420
   ggtgggacag ctagggtgga ggctgccgtt gtctttttca aatgggggac atgagggagc 480
   ctccagaatg tggtctccaa atatcacctc tcactaaggg aagggagatc agtgggggat 540
   cccaggcctg gggcaggaaa gacacataag acaaataaga aaggaacatg ataagacaga 600
   acctcttatc ctctgagtaa tcaggagcca tttaatcccc tggggccatg tgaggaagtc 660
   tgaagccact tggggagact ccccctggga aaatattcat cattggtggc cacctgtcat 720
   catcaattaa atataatgac agtggcttca aaagcattag atggcttgaa tcctagagtt 780
   caccatgaga gaagacatga attgttcacc ctggaaggat ggctgggccc cggatcttct 840
   gtgtgcaaag tggctccttc ctagagcatg aagcacactc ctgggtgaac agagcccagc 900
   tgcagacctc tgacatcaca gacactctgc agcactcatg agaaggaggt gaactgaagc 960
   ctcaaccctc tgccttcctt cccttctgag ggatgtagct attgaccatc ctatgcagct 1020
   aacattacta caagagtggc agtctgacat gggtgactct gctggtagca gctccctgtc 1080
   cttgcaagct attttgcaat gtccaatctg aatcaagtta atccccttca tgctagaatc 1140
   attttccagg aaaataaaag ctgagggaag ccctaggttc ctgctccagg aggcgtggct 1200
   gtatagcagc caggggaagt tctccagaca gctctcagct cctgcagctc ctgccttgcc 1260
   gggaggctct agagcaattg aggaaatatt caccagtgac aaccagcctg atggcacatg 1320
   cacccatcac catttgatgt catattgctc ctcatataaa cttaaacccc caaataccaa 1380
   cagcaccaac atgtaactaa gccaaagcac ttctggtgtt tctgatgcat ttgaggagac 1440
   cctgcttatt tctcgatatt agggaattag tgataatttc tatttaggtt tggtagtgat 1500
   attatagtta ttttaaggtc catgtcattc caggacagta atggagtact tacagctgaa 1560
   cagatctgat gtcttgtatt tctttctaaa tgttgtgtaa gggagaattt attttgctgt 1620
   gggtgggtcg gcatcatctg tgaattgctt attttgagtg ctgggtgatg agaacatggg 1680
   acactgttat ttttctgtct ccttatttat atgttcagag ttcttcataa tataaactta 1740
   tgacacagct cagtctttgg acagctgctg ggtgtgaagt agaccttggc cttgtaccca 1800
   gcaggcctac gtaactggcc ctccttgggt ggtcttagtt ttctcatttg tgaaatgggc 1860
   ttagacaggg actgtggcca ctagaacctt ttagccttct tctaattcag aggctgggat 1920
   ttggatcagg ggtggctgtg atcaagcacc aaggggccat ttctctgcct cattgtgact 1980
   gcagagcttc ctgtctttgc gggctctgtc cctggcctgt cgggaccacc cagcagctct 2040
   ggtgaactca gctgccacat tcctgatgtg ctggaggcag agacagggag tctaagtttg 2100
   cgtgggcact aagaacgcac agtcatccag ggcctccata gaggtttggg taggaaggct 2160
   ccaccactgt gaccaaggga gggcgaaggt cccagtgggc aggagggtct gggggagctg 2220
   agtgttcatg gatgcctgac cttgcctgta gccctctgtc tgcattcaca cagccctgac 2280
   ccagcctggc ccagctccaa ctcttgtcat gaatttgaca cggcctcctt cactcctcga 2340
   gcctgaccct gagcctgtag ccatcttatc atacacccgt aacatctttg ttcttaaatc 2400
   tcactttatg tttcataaac tacttccatg acatactagt atcttgcaga atattatggg 2460
   gcttttagag gtgccagatg gataaatgtg actttacata agctaagctt taccacagga 2520
   aattatttcc ctagtagaga acttttcatt gcctttactt ggctaacgtg cattgtcaag 2580
   tctaggaaat gcacctgtat gtttcattct ccaaggcttc tcaggacatc ctggtggcag 2640
   cacaagtatg gactcctttc ctgctaaacg gtcttgtgtg gaatggggat tttcaactgt 2700
   gttcctgctg agaccatgac attgactcat aggtgctctt cacccttact tctgcctcta 2760
   gcccacatct gggctcccca gagacccctt cacttctcct gatttccctc tccggactgt 2820
   cctgcatcct gatgctgggc catggtattt cccagtttgg caagagctac agcccaccag 2880
   atgcttgcag gacaatatga aactctggat catggattct cctgacacag agagctcacc 2940
   agagtcactg gcttgttgca tagggtcagg gatgccatgg cagggacctg gcatctgacc 3000
   aagagcggac ctatcccctg tcccggacca gcaactcttt agcccagaga ctggagcaga 3060
   cacagaaggc atgcgagaag tgccgtgtga tgcgtggtta ggtttctgtt gaaaggcaga 3120
   atctaggaga gtgtgacacc cccctggtcc cagccaggcg aggctggtgg ctcaggggta 3180
   cagtggagag ctgaggaagg ggtgtctggg tgaggaaggc cttacctgca gagatgccca 3240
   gggaactgag aaccccttca cataggcaac tttcccactg ggttcagggt tctttccacc 3300
   acaaattgag aacccaggaa aggctaggaa ttatatgtga gaaaagaaaa aatgagataa 3360
   aacaagattt ctgattgaca atgagaatat ttattgaggg tttattgagt gcagggagaa 3420
   gggcttgatg ccttggggtg ggaggagaga cccctcccct gggatcctgc agctctagtc 3480
   tcccgtggtg gggggtgagg gttgagaacc tatgaacatt ctgtaggggc cactgtcttc 3540
   tccacggtgc tcccttcatg cgtgacctgg cagctgtagc ttctgtggga cttccactgc 3600
   tcaggcgtca ggctcagata gctgctggcc gcgtacttgt tgttgctttg tttggagggt 3660
   gtggtggtct ccactcccgc cttgacgggg ctgctatctg ccttccaggc cactgtcacg 3720
   gctcccgggt agaagtcact tatgagacac accagtgtgg ccttgttggc ttgaagctcc 3780
   tcagaggagg gcgggaacag agtgaccgag ggggcagcct tgggctgacc tgcggggtgg 3840
   atgaggggca gggggtcaga gtcctggtgt ccacctgggg agcccctgac ctcagtatat 3900
   gatgaggtgt tggggtgccc ttgttcaggg gctctgtgtc tgaaggtcta tgccaggcag 3960
   aggggtcagg gtgttttcca ctatgccctg atctgcacgg ggagctgttt taccttagac 4020
   acctgaccac ctgtcctgag atctctcggg gccttggccc ccaacaccga catctgaccc 4080
   tcagcgccct gaccctgtcc cctcctgcca tcctgacaga taaagagagc tctgtgtctc 4140
   ccctgcacag cgaactctgg ggttactttt ctggcctggc tcctcagctc tgtgggatac 4200
   tggggccttt tggtccctca ggctgttcca gccaaatgtg tgttctggag cccgtgtatg 4260
   gggcaagggc ctggggggat cggagagcct gcctgctgtc ctgggctctc tcttattccg 4320
   tggagtcttt tctggcatcc accccacgtc ccggctacct ggtcatagca gtggggcttt 4380
   cacgcagtgg actctgcccc tctgatcccc ctgggcacag gggatatgtt ctgaagagac 4440
   ccaggagccc gcccttccgt cacatccact ccttttgtga cccttcccct ggctttctgg 4500
   tgtccagagt ctgcttctcc cttctccctc ctgacggggt ttcctgagtc tcaggctggc 4560
   tcagggctca gtccctggca gcccccagac gcccagggca gcccctccac ctcctacctc 4620
   attctgggtc tggatcctgg ctctgggtcc cagctctgtg attcccgagc atcagccccg 4680
   agggcactgg gtccttctaa agtctcccag gaggtgcctc cctcagaccc cttgttccca 4740
   attccccagg agatggaacc caggtaaccc aaggacaaag ctgttccgac aagaacccag 4800
   actgaggagg ccagaacaga acaaggctta gagacccagc ctgcgcctgt cagacagtca 4860
   gagagacaaa cagagagaca gagagacaaa cagagagaca gacagacaga cagacagacg 4920
   aggctcagta ggtcattcta tctagtcccc tgcccctgtg gtcagaggtg aagtggttgt 4980
   agtgacctgc tgaggcaagc aagggtctga acagggaggg cagaggtcct tgctcaggcc 5040
   tgggatgcag ggagaaaggc tgaccacaag ttgagacaag atacagaaac aaacaaaaac 5100
   agcagaaatt gtcccaagag cggggaagga gaggggagaa gagactcacc taggacggtc 5160
   agcttggtcc ctccgccgaa aaccacacgg tgaccactgc tgtcccggga gttacagtaa 5220
   tagtcagcct catcttccgg ccgcagcggc caagggcgaa ttcgcggccg ctaaattcaa 5280
   ttcgccctat agtgagtcgt attacaattc actggccgtc gttttacaac gtcgtgactg 5340
   ggaaaaccct ggcgttaccc aacttaatcg ccttgcagca catccccctt tcgccagctg 5400
   gcgtaatagc gaagaggccc gcaccgatcg cccttcccaa cagttgcgca gcctatacaa 5460
   acgaattcgc ccttagtata ccccagaact ctgcttctga gcccacagct aaggaggaac 5520
   ctccaggcct ctcttatcat aggaagggaa gtctcttcat gcaaatctac ttcctttatt 5580
   cttgtgtcgt cgttaggtgg ctctggtgag cagtggatgc aaatctgttc tccattccct 5640
   aaaacatttg tcctggttct tgtctgagcc ctgagcctga tggccttctc tgagtaattt 5700
   ctcaagatca gaagaagggc caagtgcatt ttcagattta cccactagaa ggggcctcat 5760
   aggaagcaac agtcaggctc ttcgcccttg agcattacta ggggcttgga ctcattaggg 5820
   gcctcagact aaacctcaca gtgccccctg gtgcacacag catgaatgcc attttctgca 5880
   catcactaga tcactcagaa gggctactta tgagtccata atacatgcca gatgcattag 5940
   cgagagtttt acaagtgtta cctcaagaag acttttgtaa tatagaatag gatgatgggg 6000
   agccttgtgc ttcaagccca tctttattag tgagtgaata ttaggaggtt aaatgttttt 6060
   cccaagtcct ccagacaata gggagtggag tcaagattcc acccaggaga tgcacctcag 6120
   agcccgacct tccagcccct tcctgcaccg cctcctgcac cctgctccat ctcctccttc 6180
   tccctcatcc aaggtctttg gggtccctct tgtttttgct agagctccca caagaagata 6240
   ggaaagaatt agtgaccgaa atggcagaaa catattttct cacagttctg aagctggaaa 6300
   acccaagata aaggtggtag tgggtttggt ttcccctgag gactctctcc ttggcttgca 6360
   ggttgctacc tccttgctga atcctcacct ggtcttttct ctgcacgctc acctctggtg 6420
   cctcatttct gtgtgtgcaa atttcttctt cctaggagga catcagtcca atttgagaag 6480
   aatccaccct aattttttca ttttcactta gtcacctctt taaagaccct atgcacaatt 6540
   tcagtcacat attgtgtgtt acagcttcaa tgcatacatt ttgggggact taattcagcc 6600
   aataaccccc caccctctgg actccaaaaa actcatgtct ttctcacttg taaaaacatt 6660
   caactcattc caacagcgca agtcctaaac taactcagca cctactctaa gacccaactc 6720
   tcatttagat atcacccaaa tcaagtgtgg gtgattatcc aggatgattc atcctgaggg 6780
   tttaaaggaa aacaatttgg ggggtttcct ctccagtctc ctgttatcat ccaggttcca 6840
   gaaaagtaaa ctcctacttc aataagattg aaataaaaaa gtgagtcaac atcagcaaca 6900
   tttttgagtc agatgtgaaa aattcacatc tgtgcacttt taaaataatt tagagtatag 6960
   aataatttaa atgccataaa attgaatttt gtgatcagta gccatgggct atctctgaca 7020
   gcatttggaa aagagctata gttgatacag aaaatataac ccaaaaaaca agagttgttt 7080
   ttagagcaga atatttccaa gttaactaag ataaatcgta cacagaaata atataatcca 7140
   aagaaaatta atttcttgta ggcatattaa attactaaat ccaattttag cattgagcat 7200
   caacattttg gtatgcatat aaagccacct ctcaatggtt atataaaagg gcagttgttt 7260
   cctacttcat gttacagttt atttgtggct taatggtgct ttgatagtca gagagaaggt 7320
   gctccagaag atgcttcctt tacctaaaag agggaaaata ttcttttaca taaaacgata 7380
   atttgcagga taacatattt ccactctcat catccctgtt cttgtcatgg caacttagtt 7440
   ggaggactta cagttaaaat ctcaaactca gccaggcacg gtagttcatg cctgtaatcc 7500
   cagcactttg ggaggccgag acgggtggat catgaggtca ggagattgac accatcctgg 7560
   tcaacattgt gaaaccatgt ctctacaaaa aatacaaaaa ttagcctggc atgatggcga 7620
   gagcttgtaa tcccagctac ttgggaggct gaggcaggag aatttcttga acccgggagg 7680
   tggatgttgc agtgagccgc gattgtgcca ctgcactcca gcctggcaac agagtgagac 7740
   tccatctcaa aaataaataa ataaataaat aaattttaaa aacatctcaa actctcttct 7800
   ttattccatt gttgcattct gagtttaacg atctacttgc tttataaaaa tttgcaatac 7860
   atttaaaagt tcaacttcat ttatcatttg ttaatgatgc tcagaacacc aaccatcttg 7920
   tgtgtttttg gtttcagcta caaggctgat tttaagacct tttttctctc agcctacact 7980
   ggatcactcc cgacttggtg caagcttcct tgcccatttg cccctccctc tgcctctaat 8040
   ttttcacctc ggaggaatct ctattcctga tatactgaag ctctcacaaa acagagcaaa 8100
   tggatgaaga tgaaagtcat gagcttgtga cacagggctg tcttgagcag acacaagatc 8160
   caatcaaaca catggtacag gtaggttctt atccaaagaa agacctcagg cccctagagg 8220
   aggtgaatta cagtgtccct cacaaagaga tctccacata gaacatatgc gggtgcaatg 8280
   ttcaaacttc aaataaccct agaaaccaga gaccgttaaa ccacatcctg tgggacatcc 8340
   ctggcctgcc accaattttt ttaatggccc cagaactgat agtggttttt atatgtttta 8400
   atgattacgt tgtagatggt gtatcagaac atacatatta tcccaacgtt gtcttgtgca 8460
   tggcaaagac taaaatacct cttaccgagg cattgaagaa acggtttacc aaatcctaaa 8520
   acataatgag cactgaaata tttgagtaaa tgctcaacct caagcaaaat aggagaaatc 8580
   aaaaataaat gtgtaacaag ataactttgt atatctgata ggttggcaca aacttgcact 8640
   ttcaccaatg aattccaatc aatatttagg gctggtattg taaactggta gaactggagg 8700
   ggaatatgaa aacaatggat aaaagttaaa aatgcacatc cttttgaccc agcaattcca 8760
   cttctggaaa tttatcctat ggtgatatgc acacatgaac tcaaaggagt ctgcacaggg 8820
   atgcttgctg cagtgtgatt tgtaagatca aaatcatggt gataaccatt agaggctgca 8880
   tggctaaaat cctactacag aatactctac agcgttagaa tgaatgggca gtgctctatg 8940
   cctatgacgc cccaggaaag aataagaaat ggtgcaggta tgggatggcc tttgacaagg 9000
   gggactggga caggtgcctt acacttcact ctctcctttt cctctgagat tttccacatc 9060
   agacaaacag gttagggaca gtggaagccc ctcaggctgg gcctggccac ctgctctggc 9120
   ctctgaatgc agcctggcct gacagcttgg ctgcaacttc atgaccaacc cagaaccaga 9180
   accacaaaac taagctgctc tcaattccag atgcaccgaa gtgacaagat cacaaacacg 9240
   tgttctctta ccatgctaaa tattagggag caatttttat gcaacaatag acaataaata 9300
   caatttggtt tttgaaatat attttcttgt ggatatttca ggtacacaac atgttgatat 9360
   agtctaaaca cataataaaa tggttacttt gtttttaata aaacctattt aatcaaagtt 9420
   tatgaagcct aaattatata taataatggc tatgtctacc aattgactca taaaatttaa 9480
   atattggatg caactaatgt ctgaatattt attaaattca ttctcacctt tagtagatta 9540
   agaaagattt cagctacaaa agtattgaca cccatggaaa ttacaaatgc tggatgtcaa 9600
   tgaggaagaa ggaatggact cacgttaact cttccctgtg gggccatttt ggaaactccc 9660
   aacctatagg gacaccatcc acttttgggg agtttccagg acagggcccc agcgaattga 9720
   gacgggagga ttttgaggga gcactgagcc tgtcacgcaa aagggcgaat tcgcggccgc 9780
   taaattcaat tcgccctata gtgagtcgta ttacaattca ctggccgtcg ttttacaacg 9840
   tcgtgactgg gaaaaccctg gcgttaccca acttaatcgc cttgcagcac atcccccttt 9900
   cgccagctgg cgtaatagcg aagaggcccg caccgatcgc ccttcccaac agttgcgcag 9960
   cctatacgta cggcagttta aggtttacac ctataaaaga gagagccgtt atcgtctgtt 10020
   tgtggatgta cagagtgata ttattgacac gccggggcga cggatggtga tccccctggc 10080
   cagtgcacgt ctgctgtcag ataaagtctc ccgtgaactt tacccggtgg tgcatatcgg 10140
   ggatgaaagc tggcgcatga tgaccaccga tatggccagt gtgccggtct ccgttatcgg 10200
   ggaagaagtg gctgatctca gccaccgcga aaatgacatc aaaaacgcca ttaacctgat 10260
   gttctgggga atataaatgt caggcatgag attatcaaaa aggatcttca cctagatcct 10320
   tttcacgtag aaagccagtc cgcagaaacg gtgctgaccc cggatgaatg tcagctactg 10380
   ggctatctgg acaagggaaa acgcaagcgc aaagagaaag caggtagctt gcagtgggct 10440
   tacatggcga tagctagact gggcggtttt atggacagca agcgaaccgg aattgccagc 10500
   tggggcgccc tctggtaagg ttgggaagcc ctgcaaagta aactggatgg ctttctcgcc 10560
   gccaaggatc tgatggcgca ggggatcaag ctctgatcaa gagacaggat gaggatcgtt 10620
   tcgcatgatt gaacaagatg gattgcacgc aggttctccg gccgcttggg tggagaggct 10680
   attcggctat gactgggcac aacagacaat cggctgctct gatgccgccg tgttccggct 10740
   gtcagcgcag gggcgcccgg ttctttttgt caagaccgac ctgtccggtg ccctgaatga 10800
   actgcaagac gaggcagcgc ggctatcgtg gctggccacg acgggcgttc cttgcgcagc 10860
   tgtgctcgac gttgtcactg aagcgggaag ggactggctg ctattgggcg aagtgccggg 10920
   gcaggatctc ctgtcatctc accttgctcc tgccgagaaa gtatccatca tggctgatgc 10980
   aatgcggcgg ctgcatacgc ttgatccggc tacctgccca ttcgaccacc aagcgaaaca 11040
   tcgcatcgag cgagcacgta ctcggatgga agccggtctt gtcgatcagg atgatctgga 11100
   cgaagagcat caggggctcg cgccagccga actgttcgcc aggctcaagg cgagcatgcc 11160
   cgacggcgag gatctcgtcg tgacccatgg cgatgcctgc ttgccgaata tcatggtgga 11220
   aaatggccgc ttttctggat tcatcgactg tggccggctg ggtgtggcgg accgctatca 11280
   ggacatagcg ttggctaccc gtgatattgc tgaagagctt ggcggcgaat gggctgaccg 11340
   cttcctcgtg ctttacggta tcgccgctcc cgattcgcag cgcatcgcct tctatcgcct 11400
   tcttgacgag ttcttctgaa ttattaacgc ttacaatttc ctgatgcggt attttctcct 11460
   tacgcatctg tgcggtattt cacaccgcat acaggtggca cttttcgggg aaatgtgcgc 11520
   ggaaccccta tttgtttatt tttctaaata cattcaaata tgtatccgct catgagacaa 11580
   taaccctgat aaatgcttca ataatattga aaaaggaaga gtatgagtat tcaacatttc 11640
   cgtgtcgccc ttattccctt ttttgcggca ttttgccttc ctgtttttgc tcacccagaa 11700
   acgctggtga aagtaaaaga tgctgaagat cagttgggtg cacgagtggg ttacatcgaa 11760
   ctggatctca acagcggtaa gatccttgag agttttcgcc ccgaagaacg ttttccaatg 11820
   atgagcactt ttaaagttct gctatgtggc gcggtattat cccgtattga cgccgggcaa 11880
   gagcaactcg gtcgccgcat acactattct cagaatgact tggttgagta ctcaccagtc 11940
   acagaaaagc atcttacgga tggcatgaca gtaagagaat tatgcagtgc tgccataacc 12000
   atgagtgata acactgcggc caacttactt ctgacaacga tcggaggacc gaaggagcta 12060
   accgcttttt tgcacaacat gggggatcat gtaactcgcc ttgatcgttg ggaaccggag 12120
   ctgaatgaag ccataccaaa cgacgagcgt gacaccacga tgcctgtagc aatggcaaca 12180
   acgttgcgca aactattaac tggcgaacta cttactctag cttcccggca acaattaata 12240
   gactggatgg aggcggataa agttgcagga ccacttctgc gctcggccct tccggctggc 12300
   tggtttattg ctgataaatc tggagccggt gagcgtgggt ctcgcggtat cattgcagca 12360
   ctggggccag atggtaagcc ctcccgtatc gtagttatct acacgacggg gagtcaggca 12420
   actatggatg aacgaaatag acagatcgct gagataggtg cctcactgat taagcattgg 12480
   taactgtcag accaagttta ctcatatata ctttagattg atttaaaact tcatttttaa 12540
   tttaaaagga tctaggtgaa gatccttttt gataatctca tgaccaaaat cccttaacgt 12600
   gagttttcgt tccactgagc gtcagacccc gtagaaaaga tcaaaggatc ttcttgagat 12660
   cctttttttc tgcgcgtaat ctgctgcttg caaacaaaaa aaccaccgct accagcggtg 12720
   gtttgtttgc cggatcaaga gctaccaact ctttttccga aggtaactgg cttcagcaga 12780
   gcgcagatac caaatactgt ccttctagtg tagccgtagt taggccacca cttcaagaac 12840
   tctgtagcac cgcctacata cctcgctctg ctaatcctgt taccagtggc tgctgccagt 12900
   ggcgataagt cgtgtcttac cgggttggac tcaagacgat agttaccgga taaggcgcag 12960
   cggtcgggct gaacgggggg ttcgtgcaca cagcccagct tggagcgaac gacctacacc 13020
   gaactgagat acctacagcg tgagctatga gaaagcgcca cgcttcccga agggagaaag 13080
   gcggacaggt atccggtaag cggcagggtc ggaacaggag agcgcacgag ggagcttcca 13140
   gggggaaacg cctggtatct ttatagtcct gtcgggtttc gccacctctg acttgagcgt 13200
   cgatttttgt gatgctcgtc aggggggcgg agcctatgga aaaacgccag caacgcggcc 13260
   tttttacggt tcctgggctt ttgctggcct tttgctcaca tgttctttcc tgcgttatcc 13320
   cctgattctg tggataaccg tattaccgcc tttgagtgag ctgataccgc tcgccgcagc 13380
   cgaacgaccg agcgcagcga gtcagtgagc gaggaagcgg aagagcgccc aatacgcaaa 13440
   ccgcctctcc ccgcgcgttg gccgattcat taatgcagct ggcacgacag gtttcccgac 13500
   tggaaagcgg gcagtgagcg caacgcaatt aatgtgagtt agctcactca ttaggcaccc 13560
   caggctttac actttatgct tccggctcgt atgttgtgtg gaattgtgag cggataacaa 13620
   tttcacacag gaaacagcta tgaccatgat tacgccaagc tcagaattaa ccctcactaa 13680
   aggga                                                             13685